(19) Europäisches Patentamt
European Patent Office
Office européen des brevets

(11) Veröffentlichungsnummer: 0 363 585 A1

(12) # EUROPÄISCHE PATENTANMELDUNG

(21) Anmeldenummer: 89114065.9

(22) Anmeldetag: 29.07.89

(51) Int. Cl.5: C07D 471/04 , C07D 513/04 , C07D 233/86 , C07C 265/12 , C07C 331/28 , C07C 271/58 , C07C 211/46 , A01N 43/50 , A01N 43/78 , A01N 43/84 , A01N 43/86 , //(C07D471/04, 221:00,209:00)

(30) Priorität: 11.08.88 DE 3827221

(43) Veröffentlichungstag der Anmeldung: 18.04.90 Patentblatt 90/16

(84) Benannte Vertragsstaaten: BE CH DE FR GB IT LI NL

(71) Anmelder: BAYER AG

D-5090 Leverkusen 1 Bayerwerk(DE)

(72) Erfinder: Fischer, Reiner, Dr.
Nelly-Sachs-Strasse 23
D-4019 Monheim 2(DE)
Erfinder: Lindel, Hans, Dr.
Carl-Duisberg-Strasse 321
D-5090 Leverkusen 1(DE)
Erfinder: Schallner, Otto, Dr.
Noldeweg 22
D-4019 Monheim(DE)
Erfinder: Marhold, Albrecht, Dr.
Carl-Duisberg-Strasse 329
D-5090 Leverkusen 1(DE)
Erfinder: Ooms, Pieter, Dr.
Doerperhofstrasse 16
D-4150 Krefeld 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch-Gladbach 2(DE)
Erfinder: Lürssen, Klaus, Dr.
August-Kierspel-Strasse 145
D-5060 Bergisch-Gladbach 2(DE)
Erfinder: Strang, Harry, Dr.
Unterdorfstrasse 6A
D-4000 Düsseldorf 31(DE)

(54) **N-Aryl-Stickstoffheterocyclen.**

(57) Die Erfindung betrifft neue N-Aryl-Stickstoffheterocyclen der Formel (I)

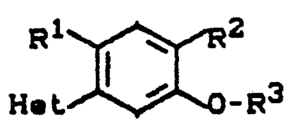

(I)

in welcher
Het für einen Heterocyclus der Formel

EP 0 363 585 A1

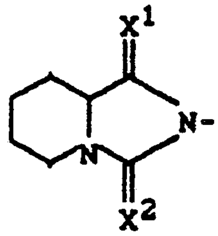 ; 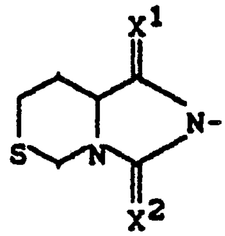 ; 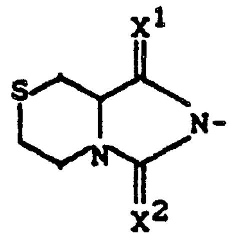

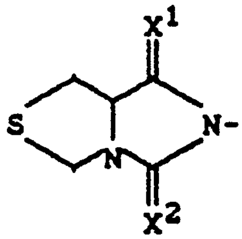 oder 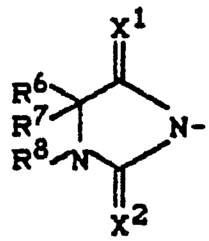 steht,

$R^1$ für Wasserstoff oder Halogen steht,
$R^2$ für Alkyl steht und
$R^3$ für jeweils gegebenenfalls substituiertes Alkyl, Alkenyl, Alkinyl oder Cycloalkyl steht oder für einen Rest
$$-\underset{\underset{O}{\|}}{C}-R^4$$
oder einen Rest $-SO_2-R^5$ steht,
wobei
$X^1$ und $X^2$ jeweils für Sauerstoff oder Schwefel stehen,
$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht,
$R^5$ für Alkyl, Halogenalkyl oder für gegebenenfalls substituiertes Aryl steht,
$R^6$ und $R^7$ entweder unabhängig voneinander jeweils für Wasserstoff, Alkyl, Alkenyl, Alkinyl oder Cycloalkyl stehen oder gemeinsam für einen zweifach verknüpften Alkandiylrest stehen und
$R^8$ für Wasserstoff, Alkyl, Alkenyl, Alkinyl, Alkoxyalkyl oder Cycloalkyl steht,
mehrere Verfahren zu ihrer Herstellung und ihre Verwendung als Herbizide und Pfalnzenwachstumsregulatoren.

# N-Aryl-Stickstoffheterocyclen

Die Erfindung betrifft neue N-Aryl-Stickstoffheterocyclen, mehrere Verfahren sowie neue Zwischenprodukte zu ihrer Herstellung und ihre Verwendung als Herbizide und Pflanzenwuchsregulatoren.

Es ist bereits bekannt, daß bestimmte N-Aryl-Stickstoffheterocyclen, wie beispielsweise das N-(2-Fluor-4-chlor-5-propargyloxy-phenyl)-$\Delta^1$-tetrahydrophthalimid oder das N-(2-Fluor-4-chlor-5-methoxycarbonylmethoxy-phenyl)-$\Delta^1$-tetrahydrophthalimid herbizide Eigenschaften besitzen (vgl. EP-A 61 741 bzw. EP-A 83 055).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Über eine pflanzenwachstumsregulierende Wirkung der vorbekannten Verbindungen ist bisher nichts bekannt.

Es wurden neue N-Aryl-Stickstoffheterocyclen der allgemeinen Formel (I),

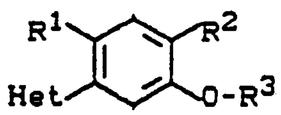

(I)

in welcher
Het für einen Heterocyclus der Formel

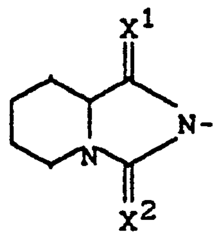
;
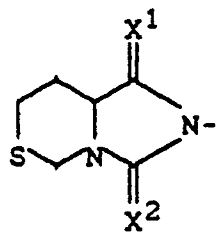
;
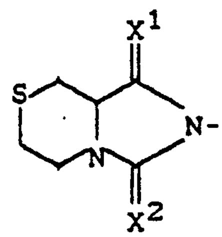
;

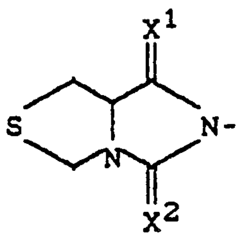
oder
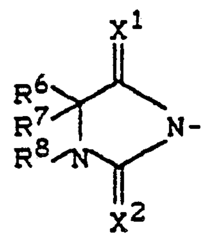
steht,

$R^1$ für Wasserstoff oder Halogen steht,
$R^2$ für Alkyl steht und
$R^3$ für jeweils gegebenenfalls substituiertes Alkyl, Alkenyl, Alkinyl oder Cycloalkyl steht oder für einen Rest

$$-\underset{\underset{O}{\|}}{C}-R^4$$

oder einen Rest $-SO_2-R^5$ steht,
wobei
$X^1$ und $X^2$ jeweils für Sauerstoff oder Schwefel stehen,
$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht,
$R^5$ für Alkyl, Halogenalkyl oder für gegebenenfalls substituiertes Aryl steht,
$R^6$ und $R^7$ entweder unabhängig voneinander jeweils für Wasserstoff, Alkyl, Alkenyl, Alkinyl oder Cycloalkyl stehen oder gemeinsam für einen zweifach verknüpften Alkandiylrest stehen und
$R^8$ für Wasserstoff, Alkyl, Alkenyl, Alkinyl, Alkoxyalkyl oder Cycloalkyl steht,

gefunden.

Weiterhin wurde gefunden, daß man die neuen N-Aryl-Stickstoffheterocyclen der allgemeinen Formel (I),

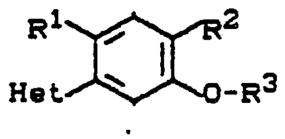
(I)

in welcher

Het für einen Heterocyclus der Formel

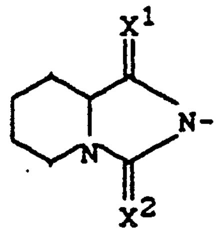
;
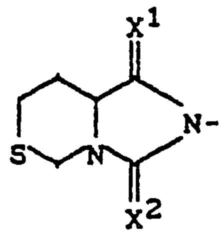
;
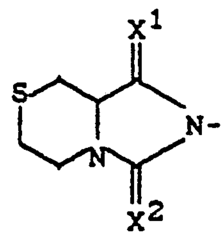
;

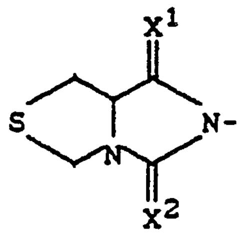
oder
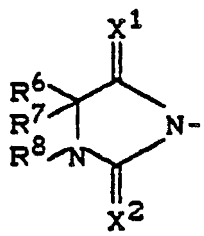
steht,

$R^1$ für Wasserstoff oder Halogen steht,

$R^2$ für Alkyl steht,

$R^3$ für jeweils gegebenenfalls substituiertes Alkyl, Alkenyl, Alkinyl oder Cycloalkyl oder für einen Rest $-\underset{\underset{O}{\|}}{C}-R^4$ oder einen Rest $-SO_2-R^5$ steht, wobei

$X^1$ und $X^2$ jeweils für Sauerstoff oder Schwefel stehen,

$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht,

$R^5$ für Alkyl, Halogenalkyl oder für gegebenenfalls substituiertes Aryl steht,

$R^6$ und $R^7$ entweder unabhängig voneinander jeweils für Wasserstoff, Alkyl, Alkenyl, Alkinyl oder Cycloalkyl stehen oder gemeinsam für einen zweifach verknüpften Alkandiylrest stehen und

$R^8$ für Wasserstoff, Alkyl, Alkenyl, Alkinyl, Alkoxyalkyl oder Cycloalkyl steht,

nach einem der im Folgenden beschriebenen Verfahren erhält:

(a) Man erhält N-Aryl-Stickstoffheterocyclen der Formel (I),

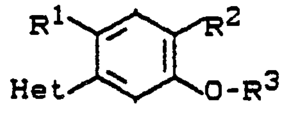
(I)

in welcher

$R^1$, $R^2$, $R^3$ und Het die oben angegebene Bedeutung haben,

wenn man Phenolderivate der Formel (II),

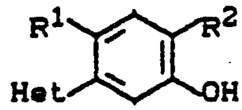

(II)

in welcher
$R^1$, $R^2$ und Het die oben angegebene Bedeutung haben,

(α) mit Alkylierungsmitteln der Formel (IIIa),

$R^{3-1}$-$E^1$ (IIIa)

in welcher
$R^{3-1}$ für jeweils gegebenenfalls substituiertes Alkyl, Alkenyl, Alkinyl oder Cycloalkyl steht und
$E^1$ für eine elektronenanziehende Abgangsgruppe steht, oder

(β) mit Acylierungsmittel der Formel (IIIb),

$R^4-\underset{\underset{O}{\|}}{C}-E^2$ (IIIb)

in welcher
$R^4$ die oben angegebene Bedeutung hat und
$E^2$ für eine elektronenanziehende Abgangsgruppe steht, oder

(γ) mit Sulfonylierungsmitteln der Formel (IIIc),

$R^5$-$SO_2$-$E^3$ (IIIc)

in welcher
$R^5$ die oben angegebene Bedeutung hat und
$E^3$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ia),

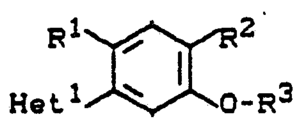

(Ia)

in welcher
$Het^1$ für einen Heterocyclus der Formel

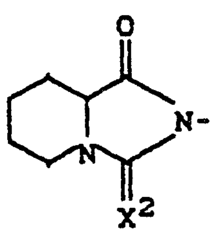 ; 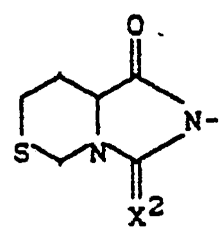 ; 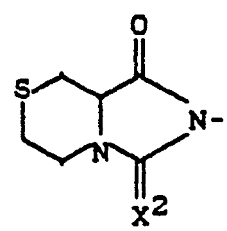 ;

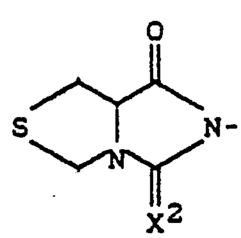 oder 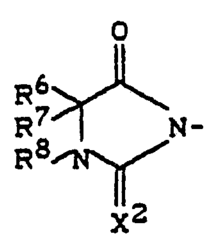

steht und
$R^1$, $R^2$, $R^3$, $R^6$, $R^7$, $R^8$ und $X^2$ die oben angegebene Bedeutung haben,

alternativ auch, wenn man Carbonsäureester der Formel (IV),

$R^9$-COOR$^{10}$ (IV)

in welcher

$R^9$ für einen Rest der Formel

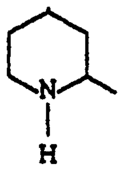 ; 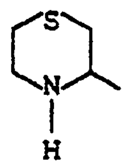 ; 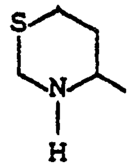 ; 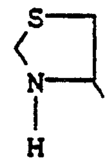

oder

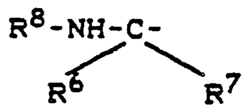

steht und

$R^{10}$ für Alkyl steht, wobei

$R^6$, $R^7$ und $R^8$ die oben angegebene Bedeutung haben,

.oder deren Säureadditionssalze,

(α) mit Iso(thio)cyanaten der Formel (V),

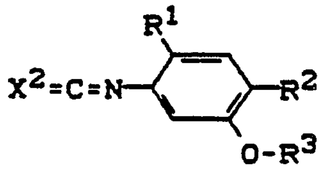

(V)

in welcher

$R^1$, $R^2$, $R^3$ und $X^2$ die oben angegebene Bedeutung haben oder

(ß) mit Carbamaten der Formel (VI),

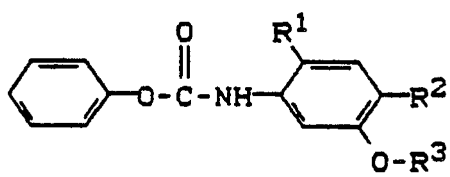

(VI)

in welcher

$R^1$, $R^2$ und $R^3$ die oben angegebene Bedeutung haben,

gegebenenfalls in Gegenwart eines Verdünnungsmittels und gegebenenfalls in Gegenwart eines Reaktionshilfsmittels umsetzt;

(c) man erhält N-Aryl-Stickstoffheterocyclen der Formel (Ib),

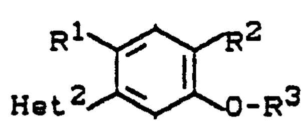

(Ib)

in welcher

$Het^2$ für einen Heterocyclus der Formel

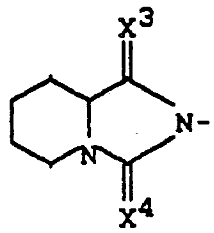 ; 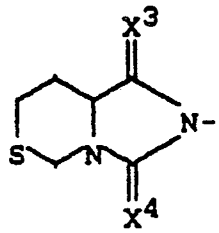 ; 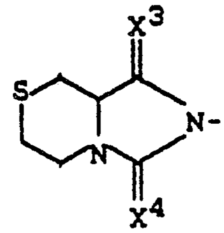 ;

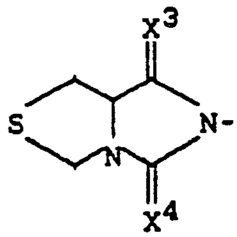 oder 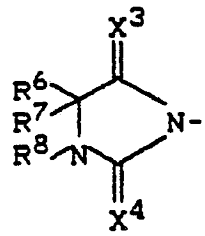 steht und

$R^1$, $R^2$, $R^3$, $R^6$, $R^7$ und $R^8$ die oben angegebene Bedeutung haben,
wobei
$X^3$ und $X^4$ jeweils für Sauerstoff oder Schwefel stehen, mit der Bedingung, daß mindestens einer der Reste $X^3$ oder $X^4$ für Schwefel steht,
wenn man die mit Hilfe der Verfahren (a) oder (b) erhältlichen N-Arylstickstoffheterocyclen der Formel (Ia),

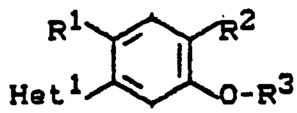 (Ia)

in welcher
$Het^1$ für einen Heterocyclus der Formel

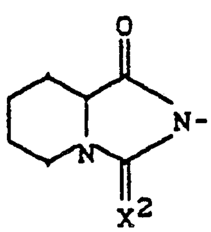 ; 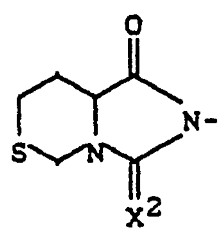 ; 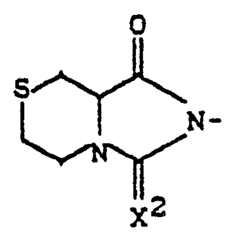 ;

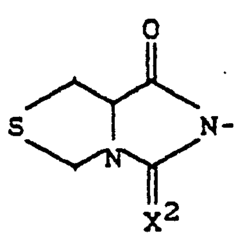 oder 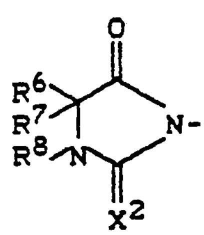 steht und

$R^1$, $R^2$, $R^3$, $R^6$, $R^7$ und $R^8$ die oben angegebene Bedeutung haben,
mit "Lawessons-Reagenz" der Formel (VII)

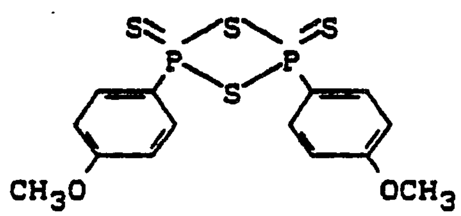

(VII)

gegebenenfalls in Gegenwart eines Verdünnungsmittels umsetzt.

Schließlich wurde gefunden, daß die neuen N-Aryl-Stickstoffheterocyclen der allgemeinen Formel (I) herbizide und pflanzenwachstumsregulierende Eigenschaften besitzen.

Überraschenderweise zeigen die erfindungsgemäßen N-Aryl-Stickstoffheterocyclen der allgemeinen Formel (I) neben einer erheblich besseren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise das N-(2-Fluor-4-chlor-5-propargyloxyphenyl)-$\Delta^1$-tetrahydrophthalimid oder das N-(2-Fluor-4-chlor-5-methoxycarbonylmethoxy-phenyl)-$\Delta^1$-tetrahydrophthalimid, welche chemisch und wirkungsmäßig naheliegende Verbindungen sind. Darüber hinaus zeigen die erfindungsgemäßen Verbindungen der Formel (I) unerwarteterweise zusätzlich eine pflanzenwachstumsregulierende Wirkung.

Die erfindungsgemäßen N-Aryl-Stickstoffheterocyclen sind durch die Formel (I) allgemein definiert.

Bevorzugt sind Verbindungen der Formel (I), bei welchen

Het für einen Heterocyclus der Formel

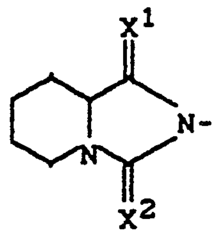 ; 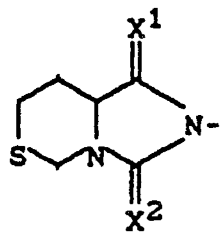 ; 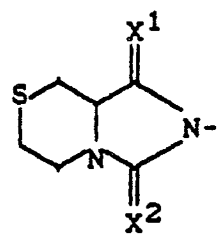 ;

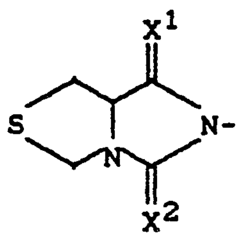 oder 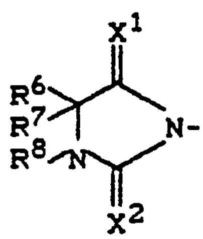 steht,

$R^1$ für Wasserstoff, Fluor, Chlor oder Brom steht,

$R^2$ für geradkettiges oder verzweigtes Alkyl mit 1 bis 4 Kohlenstoffatomen steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen; $R^3$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen

Alkylteilen steht, weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettiges oder verzweigten Alkylteil steht, wobei als Substituenten infrage kommen:
Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; und schließlich für einen Rest $-\underset{\underset{O}{\|}}{C}-R^4$
oder für einen Rest $-SO_2-R^5$ steht, wobei
$X^1$ und $X^2$ jeweils für Sauerstoff oder Schwefel stehen,
$R^4$ für jeweils geradkettiges oder verzweigtes Alkyl, Alkoxyalkyl, Halogenalkyl, Alkoxy, Alkoxyalkoxy, Halogenalkoxy, Halogenalkoxyalkoxy oder Alkoxyalkylamino mit jeweils 1 bis 6 Kohlenstoffatomen in den einzelnen Alkyl-bzw. Alkoxyteilen und gegebenenfalls 1 bis 13 gleichen oder verschiedenen Halogenatomen steht,
$R^5$ für jeweils geradkettiges oder verzweigtes Alkyl oder Halogenalkyl mit jeweils 1 bis 6 Kohlenstoffatomen und im Fall des Halogenalkyl mit 1 bis 13 gleichen oder verschiedenen Halogenatomen steht und außerdem für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio oder Alkoxycarbonyl mit jeweils 1 bis 4 Kohlen stoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
$R^6$ und $R^7$ entweder unabhängig voneinander jeweils für Wasserstoff, für jeweils geradkettiges oder verzweigtes Alkyl mit 1 bis 6 Kohlenstoffatomen, Alkenyl oder Alkinyl mit jeweils 2 bis 6 Kohlenstoffatomen oder für Cycloalkyl mit 3 bis 7 Kohlenstoffatomen stehen oder gemeinsam für einen zweifach verknüpften geradkettigen oder verzweigten Alkandiylrest mit 2 bis 12 Kohlenstoffatomen stehen und
$R^8$ für Wasserstoff, für jeweils geradkettiges oder verzweigtes Alkyl mit 1 bis 6 Kohlenstoffatomen Alkenyl mit 2 bis 6 Kohlenstoffatomen, Alkinyl mit 2 bis 6 Kohlenstoffatomen, Alkoxyalkyl mit jeweils 1 bis 4 Kohlenstoffatomen in den einzelnen Alkylteilen oder für Cycloalkyl mit 3 bis 7 Kohlenstoffatomen steht.

Besonders bevorzugt sind Verbindungen der Formel (I), bei welchen
Het für einen Heterocyclus der Formel

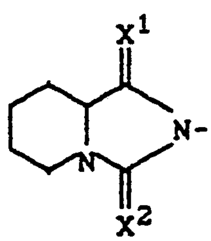 ; 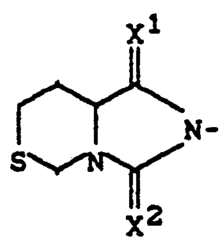 ; 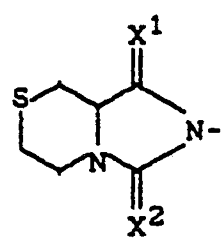 ;

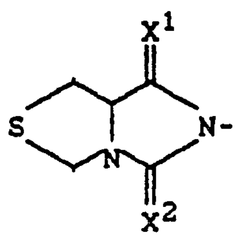 oder 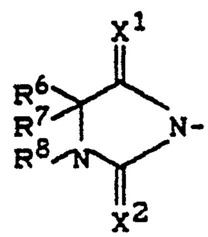 steht,

$R^1$ für Wasserstoff, Fluor oder Chlor steht,
$R^2$ für Methyl oder Ethyl steht,
$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio und außerdem für einen Rest $-\underset{\underset{O}{\parallel}}{C}-R^4$
oder für einen Rest Rest $-SO_2-R^5$ steht, wobei
$X^1$ und $X^2$ jeweils für Sauerstoff oder Schwefel stehen,
$R^4$ für jeweils geradkettiges oder verzweigtes Alkyl, Alkoxyalkyl, Halogenalkyl, Alkoxy, Alkoxyalkoxy, Halogenalkoxy, Halogenalkoxyalkoxy oder Alkoxyalkylamino mit jeweils 1 bis 3 Kohlenstoffatomen in den einzelnen Alkyl-bzw. Alkoxyteilen und gegebenenfalls 1 bis 7 gleichen oder verschiedenen Halogenatomen insbesondere Fluor, Chlor oder Brom steht,
$R^5$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein-bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy n- oder i-Propoxy, Methylthio, Ethylthio, Methoxycarbonyl, Ethoxycarbonyl, Trifluormethyl, Trifluormethoxy oder Trifluormethylthio,
$R^6$ und $R^7$ entweder unabhängig voneinander jeweils für Wasserstoff, Methyl, Ethyl, n- oder i-Propyl, n-, i-, s- oder t-Butyl, für Allyl, Propargyl, Cyclopropyl, Cyclopentyl oder Cyclohexyl stehen oder gemeinsam für einen zweifach verknüpften Alkandiyl mit 2 bis 8 Kohlenstoffatomen stehen und
$R^8$ für Wasserstoff, Methyl, Ethyl, n- oder i-Propyl, n-, i-, s- oder t-Butyl, für Allyl, Propargyl, Methoxymethyl, Methoxyethyl, Methoxypropyl, Ethoxymethyl, Ethoxyethyl, Ethoxypropyl, Propoxymethyl, Propoxyethyl, Propoxypropyl, für Cyclopropyl, Cyclopentyl oder Cyclohexyl steht.

Ganz besonders bevorzugt sind Verbindungen der Formel (I), bei welchen
Het für einen Heterocyclus der Formel

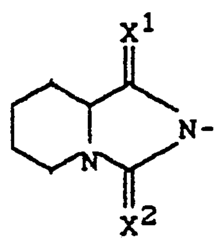 ; 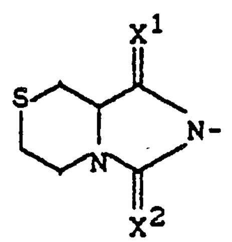 ; 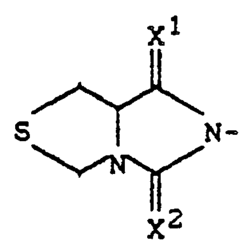

oder 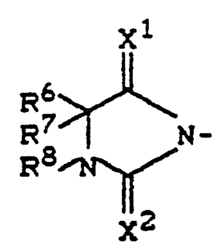 steht,

$R^1$ für Wasserstoff oder Fluor steht,
$R^2$ für Methyl steht,
$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für

jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl, Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht oder für jeweils gegebenenfalls ein- bis dreifach gleich oder verschieden substituiertes Benzyl oder Phenylethyl steht, wobei als Substituenten jeweils in Frage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propyl, Methylthio, Ethylthio, Methoxycarbonyl, Ethoxycarbonyl, Trifluormethyl, Trifluormethoxy oder Trifluormethylthio und schließlich für einen Rest $-\underset{\underset{O}{\|}}{C}-R^4$ oder für einen Rest $-SO_2-R^5$ steht, wobei

$R^4$ für jeweils geradkettiges oder verzweigtes Alkyl, Alkoxyalkyl, Halogenalkyl, Alkoxy, Alkoxyalkoxy, Halogenalkoxy, Halogenalkoxyalkoxy oder Alkoxyalkylamino mit jeweils 1 bis 3 Kohlenstoffatomen in den einzelnen Alkyl- bzw. Alkoxyteilen und gegebenenfalls 1 bis 5 gleichen oder verchiedenen Halogenatomen, insbesondere Fluor oder Chlor steht,

$R^5$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

$R^6$ und $R^7$ entweder unabhängig voneinander jeweils für Wasserstoff, Methyl, Ethyl, Allyl oder Propargyl stehen oder gemeinsam für 1,2-Ethandiyl, 1,3-Propandiyl, 1,4-Butandiyl oder 1,5-Pentandiyl stehen und

$R^8$ für Wasserstoff, Methyl, Ethyl, Allyl, Propargyl, Methoxyethyl, Ethoxyethyl, Methoxypropyl, Ethoxypropyl oder für Cyclohexyl steht.

Im einzelnen seien außer den bei den Herstellungsbeispielen genannten Verbindungen die folgenden N-Aryl-Stickstoffheterocyclen der allgemeinen Formel (I) genannt:

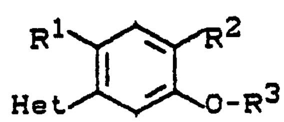

(I)

| Het | $R^1$ | $R^2$ | $R^3$ |
|---|---|---|---|
| 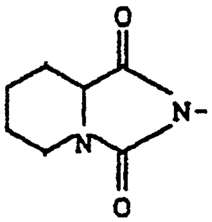 | F | $CH_3$ | $-CH_2-CN$ |
| 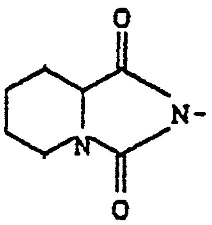 | H | $CH_3$ | $CH_3$<br>\|<br>$-CH-CN$ |
| 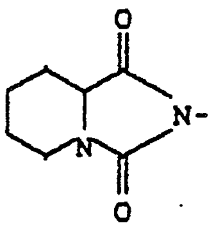 | H | $CH_3$ | O<br>‖<br>$-CH_2-C-CH_3$ |
| 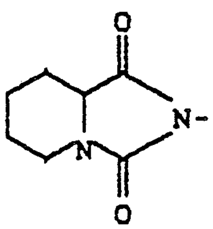 | H | $CH_3$ | $-CH_2-CH_2-O-CH_2-CF_3$ |
| 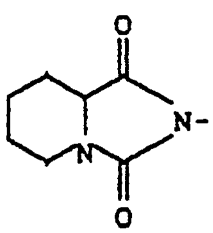 | H | $CH_3$ | $-CH_2-CH(CH_3)_2$ |

| Het | $R^1$ | $R^2$ | $R^3$ |
|---|---|---|---|
| 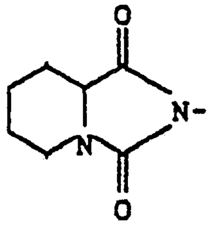 | H | $CH_3$ | $-CHF_2$ |
| 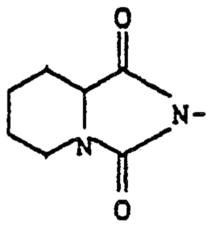 | F | $CH_3$ | 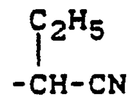 |
| 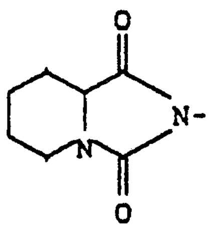 | F | $CH_3$ | 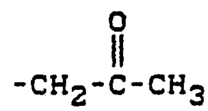 |
| 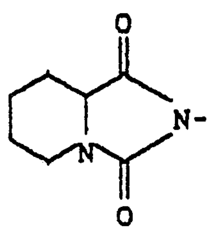 | F | $CH_3$ | 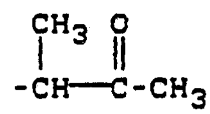 |
| 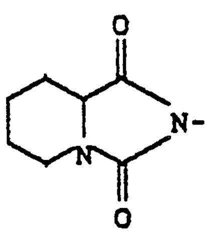 | F | $CH_3$ | 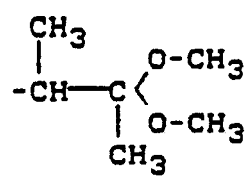 |

| Het | $R^1$ | $R^2$ | $R^3$ |
|---|---|---|---|
| 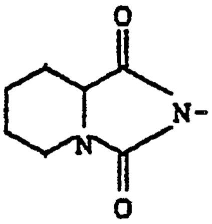 | F | $CH_3$ | 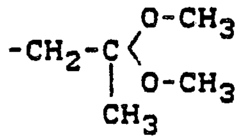 |
| 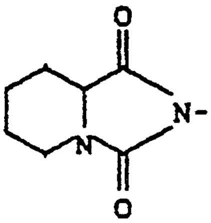 | F | $CH_3$ | $-CF_2-CHFCl$ |
| 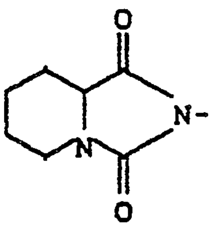 | F | $CH_3$ | $-CF_2-CHF-CF_3$ |
| 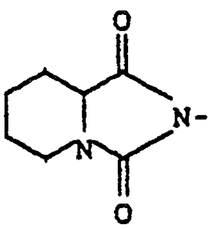 | F | $CH_3$ | 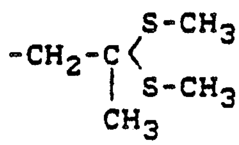 |
| 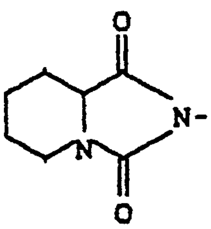 | F | $CH_3$ | $-CH(CH_2F)_2$ |

| Het | $R^1$ | $R^2$ | $R^3$ |
|---|---|---|---|
| 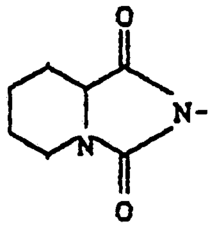 | F | $CH_3$ | $-CH_2-CH_2-O-CH_2-CF_3$ |
| 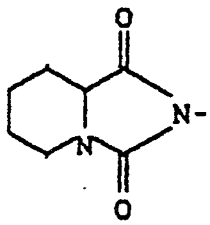 | F | $CH_3$ | 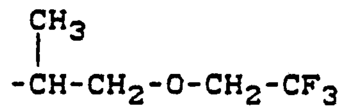 |
| 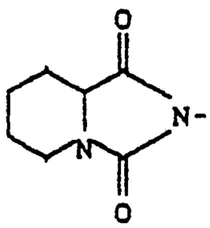 | F | $CH_3$ | 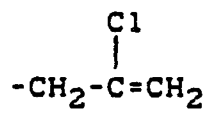 |
| 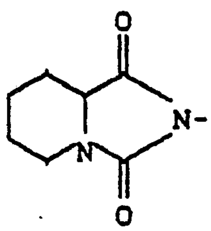 | F | $CH_3$ | $-CH_2-CH_2F$ |
| 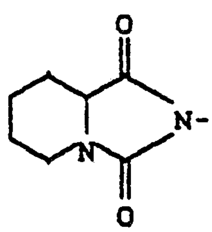 | F | $CH_3$ | $-CF_2-CHF-CF_3$ |

| Het | $R^1$ | $R^2$ | $R^3$ |
|---|---|---|---|
| 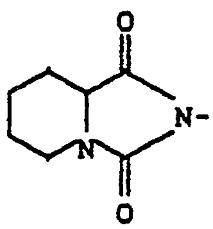 | F | $CH_3$ | $-CF_2-CHF_2$ |
| 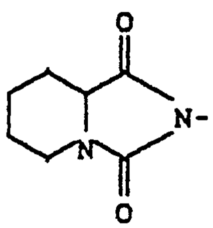 | F | $CH_3$ | $-CF_2-CHClF$ |
| 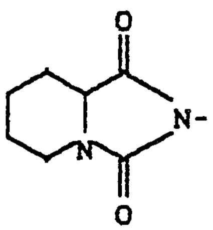 | F | $CH_3$ | $-CF_3$ |
| 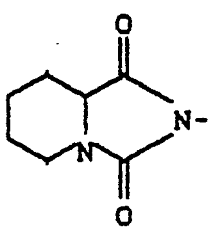 | F | $CH_3$ | $-CClF_2$ |
| 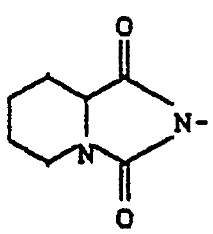 | F | $CH_3$ | $-CH_2-CH=CH-Cl$ |

| Het | $R^1$ | $R^2$ | $R^3$ |
|---|---|---|---|
| 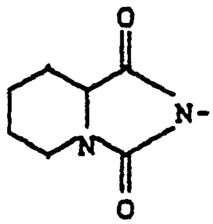 | F | $CH_3$ | $-CH_2-CH_2-Cl$ |
| 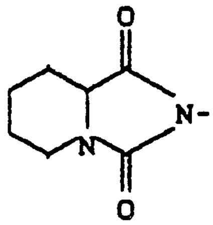 | F | $CH_3$ | $-CH_2-CH_2-O-CH_3$ |
| 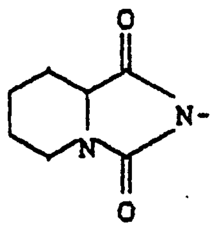 | F | $CH_3$ | $-CH_2-CH_2-O-C_2H_5$ |
| 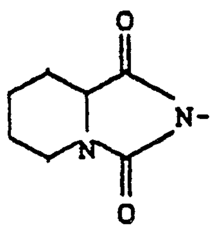 | F | $CH_3$ | $-CH_2-CH_2-O-CH(CH_3)_2$ |
| 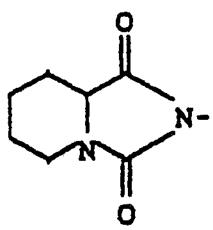 | F | $CH_3$ | $-CH_2-CH_2-O-(CH_2)_2-CH_3$ |

| Het | $R^1$ | $R^2$ | $R^3$ |
|---|---|---|---|
| 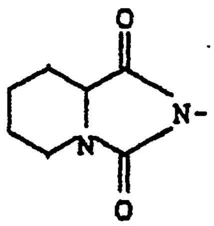 | F | $CH_3$ | $-CH_2-CH_2-O-(CH_2)_3-CH_3$ |
| 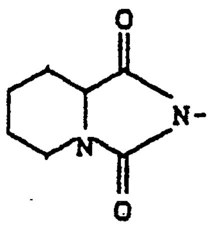 | F | $CH_3$ | $-(CH_2-CH_2-O)_2-CH_3$ |
| 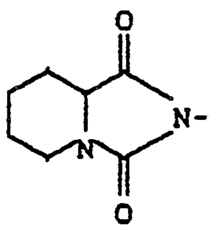 | F | $CH_3$ | $-(CH_2-CH_2-O)_2-CH(CH_3)_2$ |
| 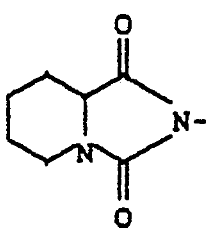 | F | $CH_3$ | $-(CH_2-CH_2-O)_2-(CH_2)_2-CH_3$ |
| 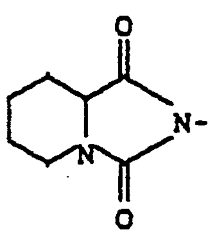 | F | $CH_3$ | $-(CH_2-CH_2-O)_2-(CH_2)_3-CH_3$ |

| Het | $R^1$ | $R^2$ | $R^3$ |
|---|---|---|---|
| 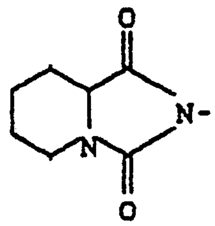 | F | $CH_3$ | $-(CH_2-CH_2-O)_2-CH_3$ |
| 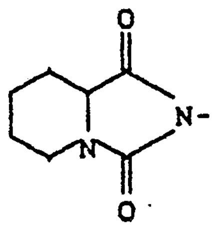 | F | $CH_3$ | $-(CH_2-CH_2-O)_2-C_2H_5$ |
| 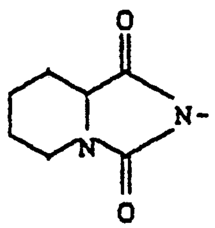 | F | $CH_3$ | $-(CH_2-CH_2-O)_2-CH_2-CH(CH_3)_2$ |
| 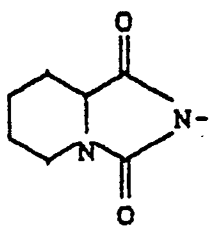 | F | $CH_3$ | $CH_3$<br>\|<br>$-CH-CH_2-O-CH_3$ |
| 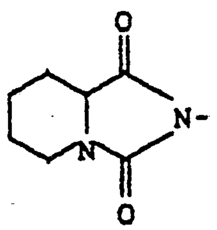 | F | $CH_3$ | $CH_3$<br>\|<br>$-CH-CH_2-O-C_2H_5$ |

| Het | $R^1$ | $R^2$ | $R^3$ |
|---|---|---|---|
| 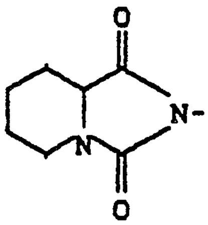 | F | $CH_3$ | 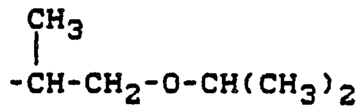 |
| 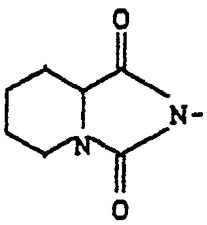 | F | $CH_3$ | 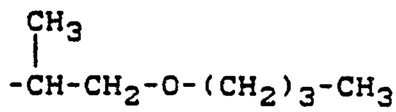 |
| 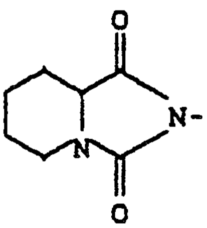 | F | $CH_3$ | 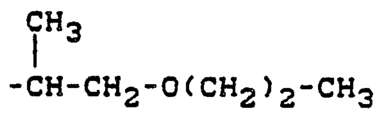 |
| 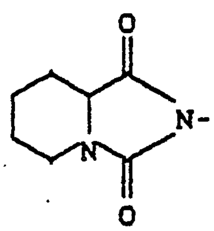 | F | $CH_3$ | 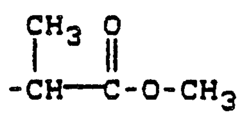 |
| 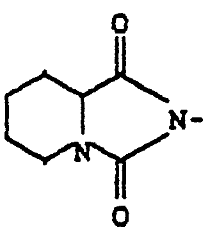 | F | $CH_3$ | 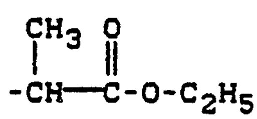 |

| Het | $R^1$ | $R^2$ | $R^3$ |
|---|---|---|---|
| 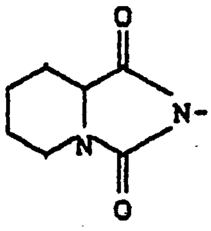 | F | $CH_3$ | 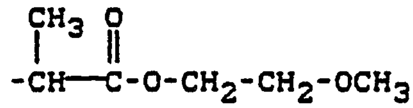 |
| 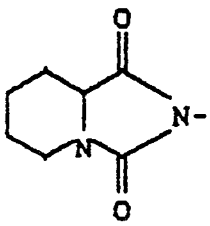 | F | $CH_3$ | 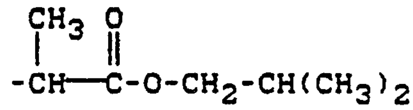 |
| 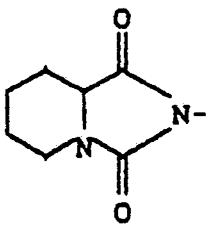 | F | $CH_3$ | 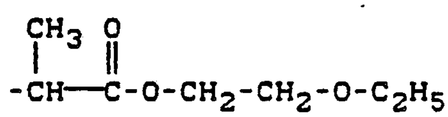 |
| 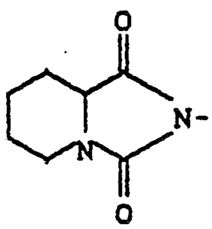 | F | $CH_3$ | 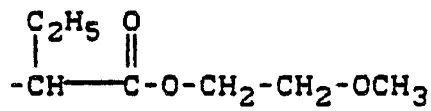 |
| 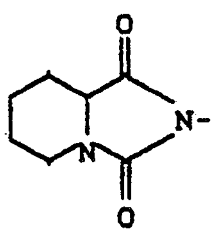 | F | $C_2H_5$ | $-CH_2-C{\equiv}CH$ |

| Het | $R^1$ | $R^2$ | $R^3$ |
|---|---|---|---|
| 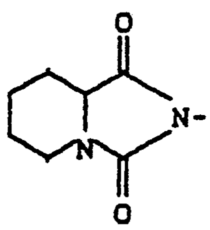 | F | $C_2H_5$ | $-CH_2-CH_2-OC_2H_5$ |
| 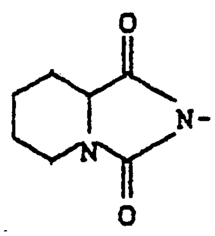 | F | $C_2H_5$ | $-(CH_2-CH_2-O)_2-CH_3$ |
| 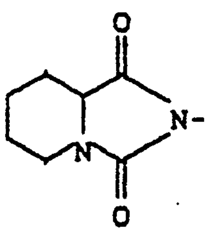 | F | $C_2H_5$ | $-CH_2-CH=CH_2$ |
| 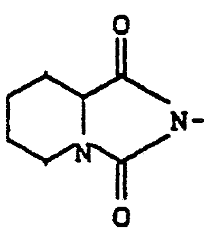 | F | $C_2H_5$ | $-CF_3$ |
| 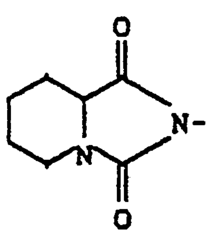 | F | $C_2H_5$ | $-CHF_2$ |

| Het | $R^1$ | $R^2$ | $R^3$ |
|---|---|---|---|
| 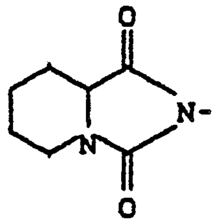 | F | $C_2H_5$ | $-CH_2-\overset{O}{\overset{\|}{C}}-O-CH_2-CH_2-OCH_3$ |
| 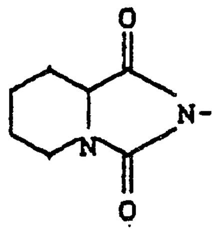 | F | $C_2H_5$ | $-\overset{CH_3}{\overset{\|}{C}}H-COOC_2H_5$ |
| 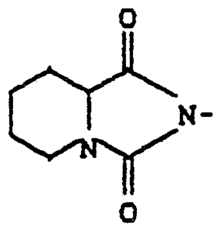 | F | $C_2H_5$ | $-CF_2-CHF-CHClF$ |
| 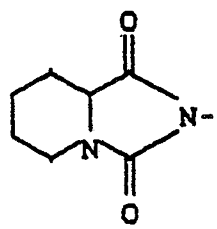 | F | $C_2H_5$ | $-CH(CH_3)_2$ |
| 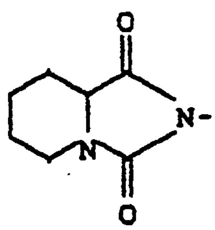 | F | $C_2H_5$ | $-CH_2-C\equiv C-CH_3$ |

| Het | $R^1$ | $R^2$ | $R^3$ |
|---|---|---|---|
| 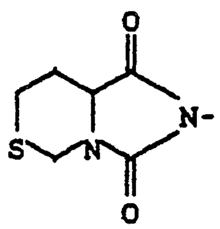 | F | $CH_3$ | $-CH(CH_3)-CH_2-O-CH_3$ |
| 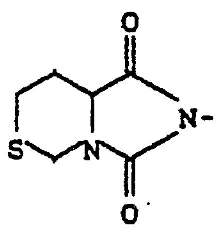 | F | $CH_3$ | $-CH_2-CH=CH_2$ |
| 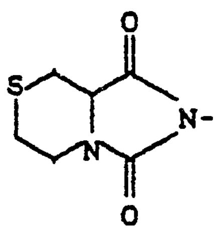 | F | $CH_3$ | $-CH_2-C\equiv CH$ |
| 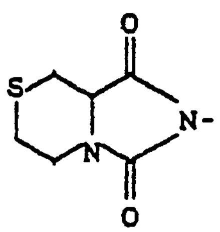 | F | $CH_3$ | $-CH(CH_3)-CH_2-OC_2H_5$ |
| 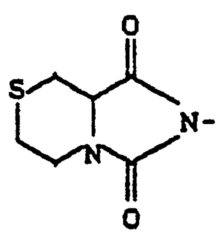 | F | $CH_3$ | $-CH(CH_3)-CH_2-O-CH(CH_3)_2$ |

| Het | $R^1$ | $R^2$ | $R^3$ |
|---|---|---|---|
| 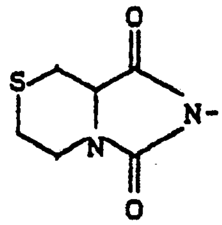 | F | $CH_3$ | 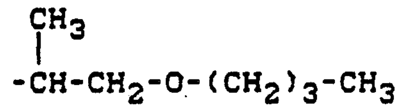 |
| 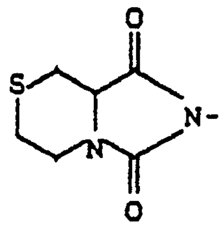 | F | $CH_3$ | 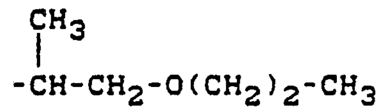 |
| 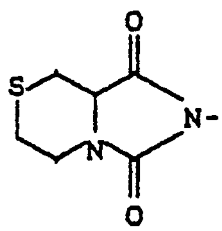 | F | $CH_3$ | 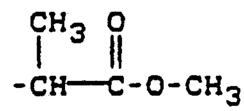 |
| 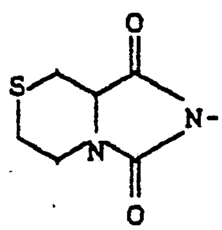 | F | $CH_3$ | 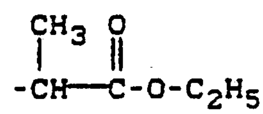 |
| 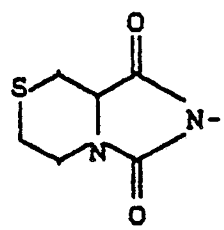 | F | $CH_3$ | 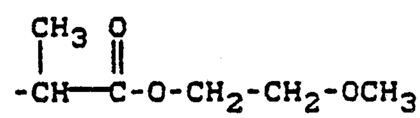 |

| Het | $R^1$ | $R^2$ | $R^3$ |
|---|---|---|---|
| 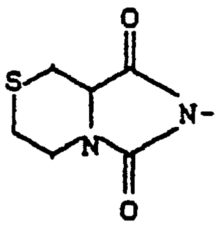 | F | $CH_3$ | 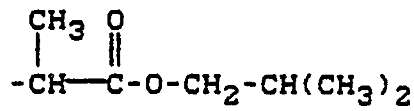 |
| 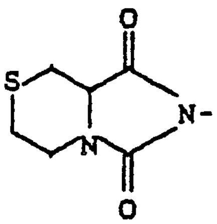 | F | $CH_3$ | 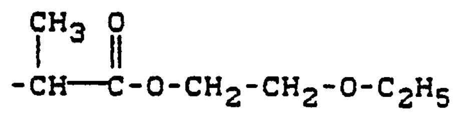 |
| 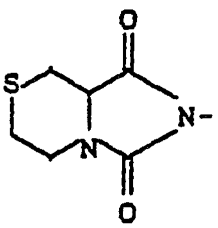 | F | $CH_3$ | 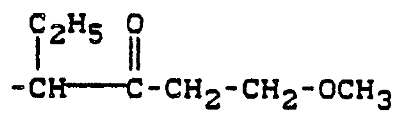 |
| 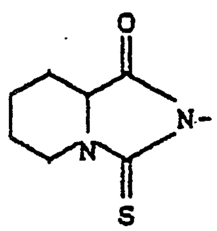 | F | $CH_3$ | $-CH_2-CH_2-O-CH_3$ |
| 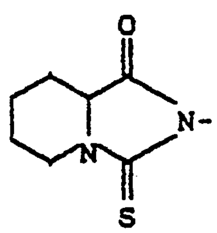 | F | $CH_3$ | $-CH_2-CH_2-O-C_2H_5$ |

| Het | $R^1$ | $R^2$ | $R^3$ |
|---|---|---|---|
| 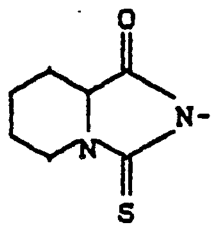 | F | $CH_3$ | $-CH_2-CH_2-O-CH(CH_3)_2$ |
| 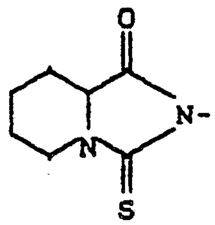 | F | $CH_3$ | $-CH_2-CH_2-O-(CH_2)_2-CH_3$ |
| 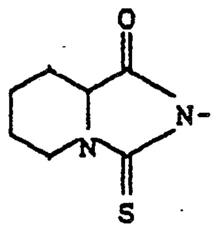 | F | $CH_3$ | $-CH_2-CH_2-O-(CH_2)_3-CH_3$ |
| 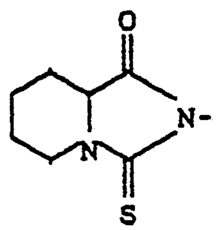 | F | $CH_3$ | $-(CH_2-CH_2-O)_2-CH_3$ |
| 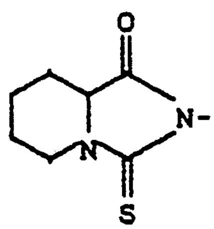 | F | $CH_3$ | $-(CH_2-CH_2-O)_2-C_2H_5$ |

| Het | $R^1$ | $R^2$ | $R^3$ |
|---|---|---|---|
| 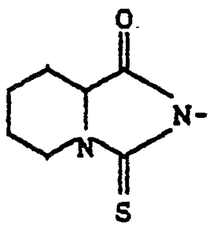 | F | $CH_3$ | $-(CH_2-CH_2-O)_2-CH(CH_3)_2$ |
| 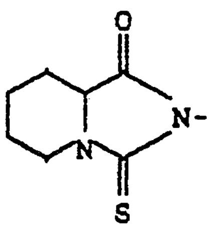 | F | $CH_3$ | $-(CH_2-CH_2-O)_2-(CH_2)_2-CH_3$ |
| 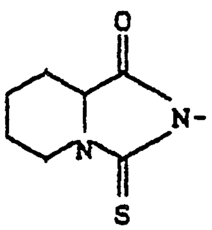 | F | $CH_3$ | $-(CH_2-CH_2-O)_2-(CH_2)_3-CH_3$ |
| 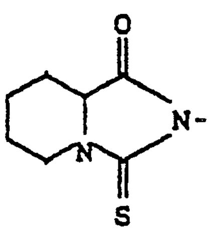 | F | $CH_3$ | $-(CH_2-CH_2-O)_2-CH_3$ |
| 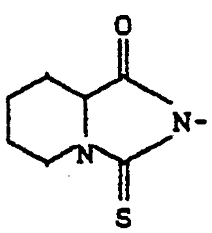 | F | $CH_3$ | $-(CH_2-CH_2-O)_2-C_2H_5$ |

| Het | $R^1$ | $R^2$ | $R^3$ |
|---|---|---|---|
| 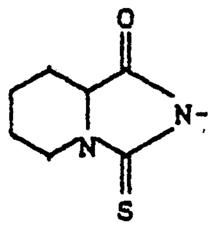 | F | $CH_3$ | $-(CH_2-CH_2-O)_2-CH_2-CH(CH_3)_2$ |
| 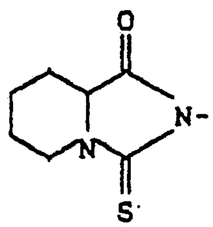 | F | $CH_3$ | $-CH(CH_2F)_2$ |
| 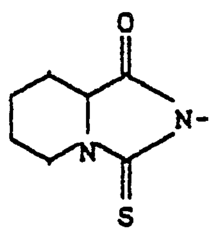 | F | $CH_3$ | $-CH_2-CH_2-O-CH_2-CH(CH_3)_2$ |
| 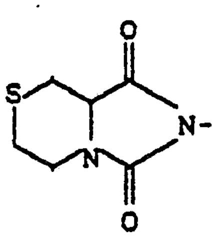 | F | $CH_3$ | 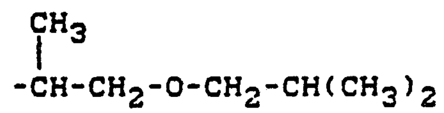 |
| 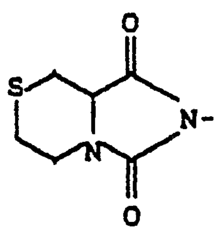 | F | $CH_3$ | 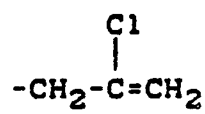 |

| Het | $R^1$ | $R^2$ | $R^3$ |
|---|---|---|---|
| 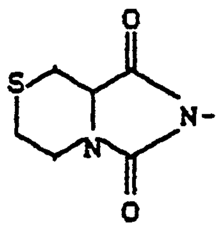 | F | $CH_3$ | $-CH_2-CH_2F$ |
| 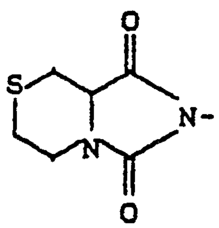 | F | $CH_3$ | $-CF_2-CHF-CF_3$ |
| 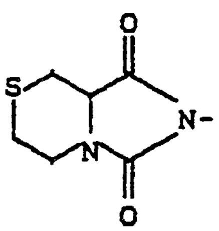 | F | $CH_3$ | $-CF_2-CHF_2$ |
| 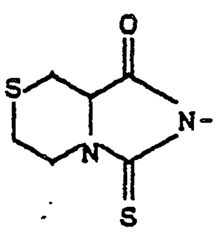 | F | $CH_3$ | $-CF_2-CHClF$ |
| 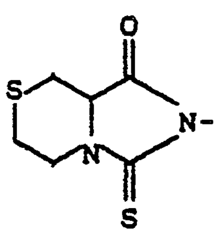 | F | $CH_3$ | $-CF_3$ |

| Het | $R^1$ | $R^2$ | $R^3$ |
|---|---|---|---|
| 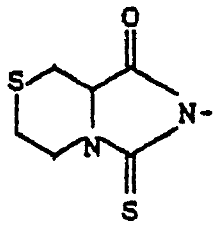 | F | $CH_3$ | $-CClF_2$ |
| 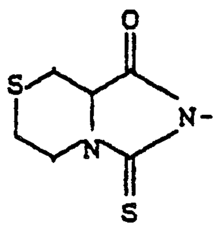 | F | $CH_3$ | $-CH_2-CH=CH-Cl$ |
| 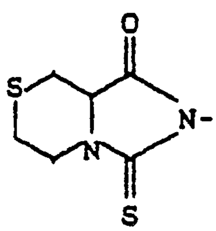 | F | $CH_3$ | $-CH_2-CH_2-Cl$ |
| 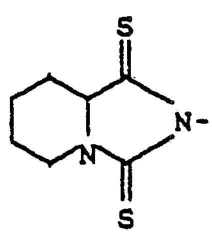 | F | $CH_3$ | $-CH_2-CN$ |
| 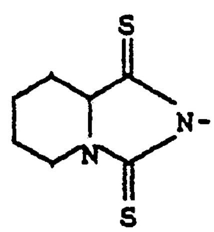 | F | $CH_3$ | 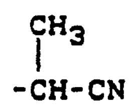 |

| Het | $R^1$ | $R^2$ | $R^3$ |
|---|---|---|---|
| 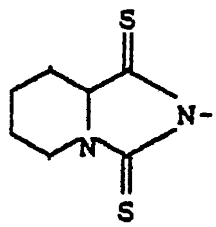 | F | $CH_3$ | 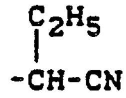 |
| 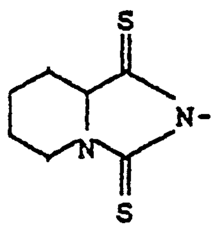 | F | $CH_3$ | 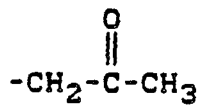 |
| 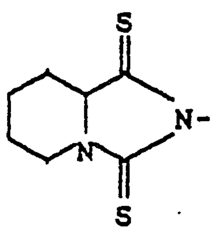 | F | $CH_3$ | 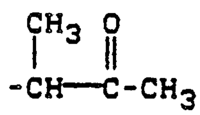 |
| 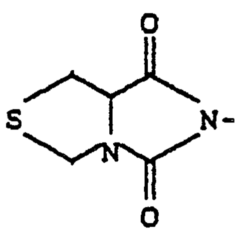 | F | $CH_3$ | 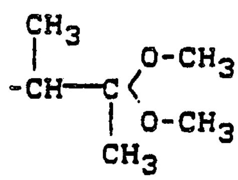 |
| 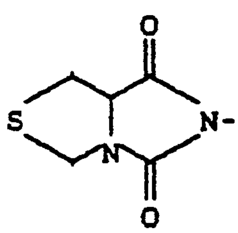 | F | $CH_3$ | 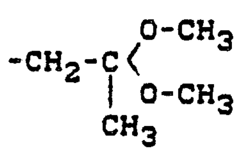 |

| Het | $R^1$ | $R^2$ | $R^3$ |
|---|---|---|---|
| 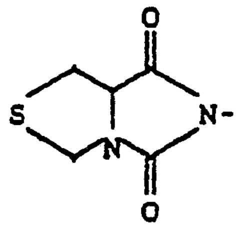 | F | $CH_3$ | $-CF_2-CHFCl$ |
| 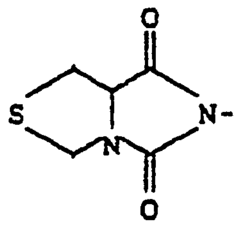 | F | $CH_3$ | $-CF_2-CHF-CF_3$ |
| 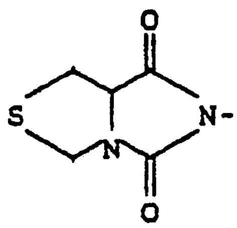 | F | $CH_3$ | $-CH_2-C(CH_3)(S-CH_3)_2$ |
| 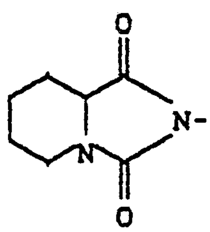 | F | $-CH(CH_3)_2$ | $-(CH_2-CH_2-O)_2-CH_3$ |
| 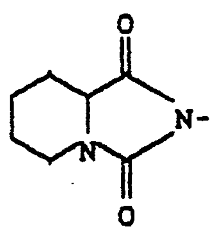 | F | $-CH(CH_3)_2$ | $-CH_2-CH=CH_2$ |

| Het | $R^1$ | $R^2$ | $R^3$ |
|---|---|---|---|
| 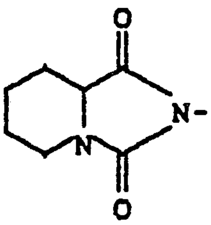 | F | $-CH(CH_3)_2$ | $-CF_3$ |
| 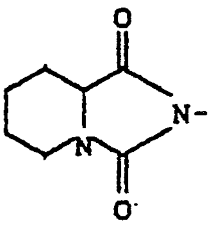 | F | $-CH(CH_3)_2$ | $-CHF_2$ |
| 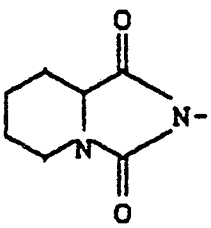 | F | $-CH(CH_3)_2$ | $-CH_2-\underset{\underset{O}{\|}}{C}-OCH_2-CH_2-OCH_3$ |
| 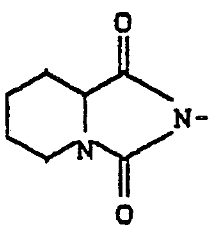 | F | $-CH(CH_3)_2$ | $-\overset{\overset{CH_3}{\mid}}{CH}-COOC_2H_5$ |
| 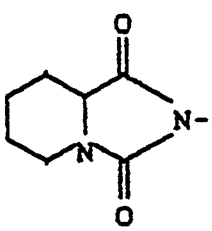 | F | $-CH(CH_3)_2$ | $-CF_2-CHF-CHClF$ |

| Het | $R^1$ | $R^2$ | $R^3$ |
|---|---|---|---|
| 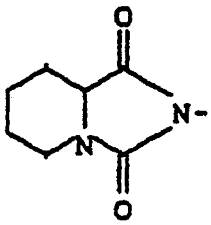 | F | $-CH(CH_3)_2$ | $-CH(CH_3)_2$ |
| 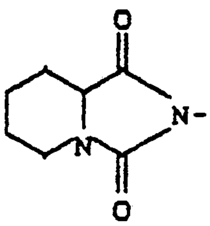 | F | $-CH(CH_3)_2$ | $-CH-C\equiv C-CH_3$ |
| 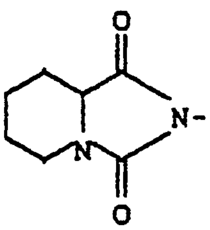 | F | $-(CH_2)_2-CH_3$ | $-CH_2-C\equiv CH$ |
| 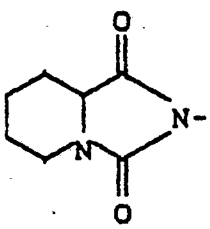 | F | $-(CH_2)_2-CH_3$ | $-CH_2-CH_2-OC_2H_5$ |
| 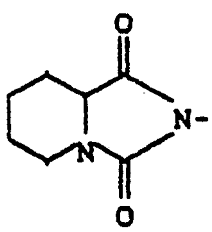 | F | $-(CH_2)_2-CH_3$ | $-(CH_2-CH_2-O)_2-CH_3$ |

| Het | $R^1$ | $R^2$ | $R^3$ |
|---|---|---|---|
| 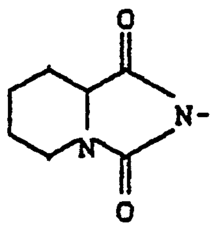 | F | $-(CH_2)_2-CH_3$ | $-CH_2-CH=CH_2$ |
| 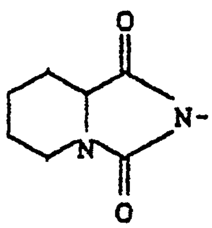 | F | $-(CH_2)_2-CH_3$ | $-CF_3$ |
| 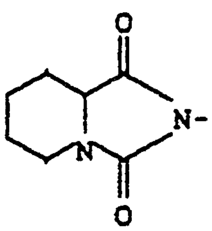 | F | $-(CH_2)_2-CH_3$ | $-CHF_2$ |
| 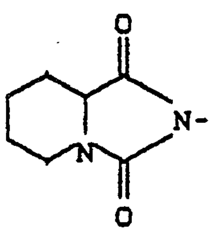 | F | $-(CH_2)_2-CH_3$ | $-CH_2-COOCH_2-CH_2-OCH_3$ |
| 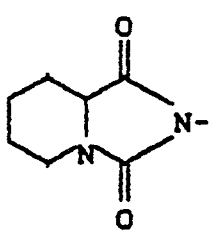 | F | $-(CH_2)_2-CH_3$ | $CH_3$<br>$-CH-COOC_2H_5$ |

| Het | $R^1$ | $R^2$ | $R^3$ |
|---|---|---|---|
| 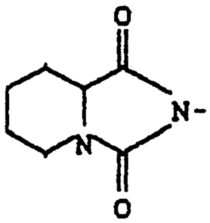 | F | $-(CH_2)_2-CH_3$ | $-CF_2-CHF-CHClF$ |
| 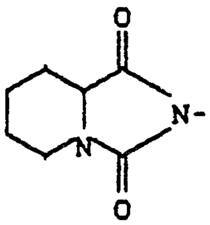 | F | $-(CH_2)_2-CH_3$ | $-CH(CH_3)_2$ |
| 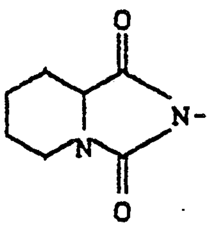 | F | $-(CH_2)_2-CH_3$ | $-CH_2-C\equiv C-CH_3$ |
| 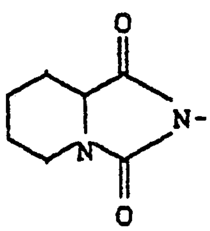 | F | $CH_3$ | $-\underset{\displaystyle CH_3}{\underset{\vert}{CH}}-CH_2-O-CH(CH_3)_2$ |
| 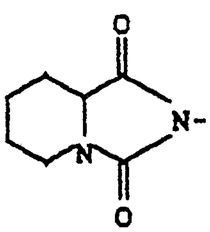 | F | $CH_3$ | $-\underset{\displaystyle CH_3}{\underset{\vert}{CH}}-CH_2-O-(CH_2)_2-CH_3$ |

| Het | $R^1$ | $R^2$ | $R^3$ |
|---|---|---|---|
| 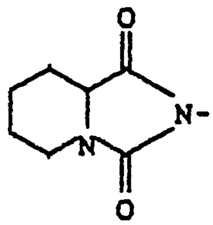 | F | $CH_3$ | $-CH-CH_2-O-C_2H_5$<br>$\vert$<br>$C_2H_5$ |
| 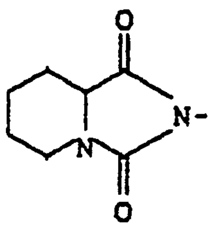 | F | $CH_3$ | $-CH_2-C-CH_3$<br>$\Vert$<br>O |
| 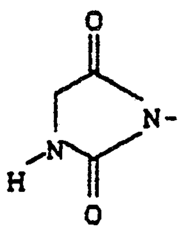 | F | $CH_3$ | $-CH(CH_3)_2$ |
| 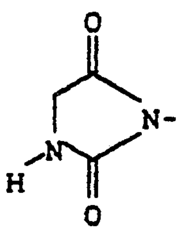 | F | $CH_3$ | $-CH_2-C{\equiv}CH$ |
| 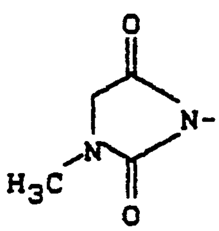 | F | $CH_3$ | $-CH(CH_3)_2$ |

| Het | $R^1$ | $R^2$ | $R^3$ |
|---|---|---|---|
| 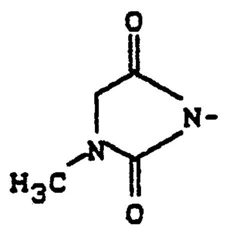 | F | $CH_3$ | $-CH_2-C\equiv CH$ |
| 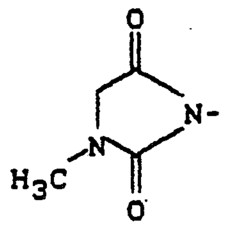 | F | $CH_3$ | $-CH_2-CH=CH_2$ |
| 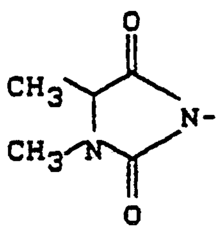 | F | $CH_3$ | $-CH(CH_3)_2$ |
| 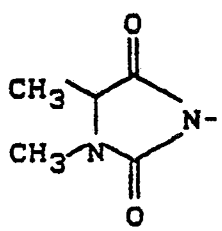 | F | $CH_3$ | $-CH_2-C\equiv CH$ |
| 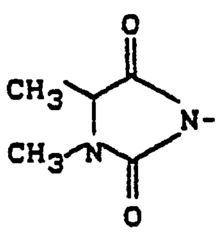 | F | $CH_3$ | $-CH_2-CH=CH_2$ |

| Het | $R^1$ | $R^2$ | $R^3$ |
|---|---|---|---|
| 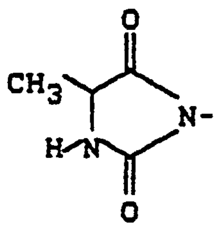 | F | $CH_3$ | $-CH(CH_3)_2$ |
| 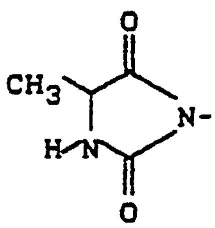 | F | $CH_3$ | $-CH_2-CH(CH_3)_2$ |
| 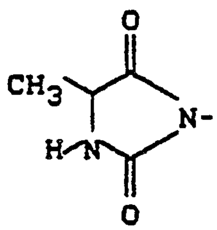 | F | $CH_3$ | $-CH_2-C\equiv CH$ |
| 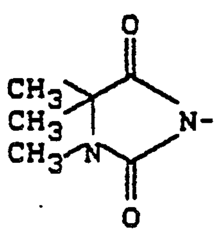 | F | $CH_3$ | $-CH(CH_3)_2$ |
| 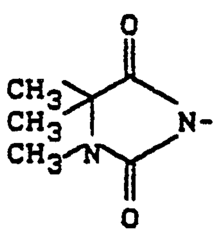 | F | $CH_3$ | $-CH_2-C\equiv CH$ |

| Het | $R^1$ | $R^2$ | $R^3$ |
|---|---|---|---|
| 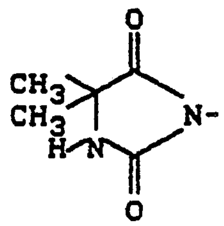 | F | $CH_3$ | $-CH(CH_3)_2$ |
| 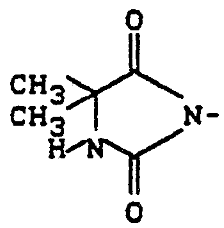 | F | $CH_3$ | $-CH_2-C\equiv CH$ |
| 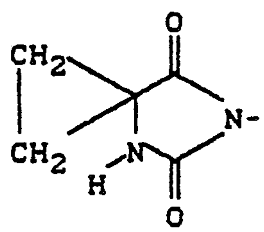 | F | $CH_3$ | $-CH(CH_3)_2$ |
| 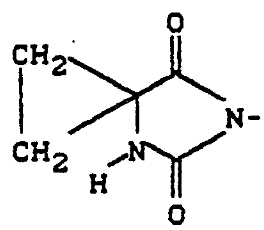 | F | $CH_3$ | $-CH_2-C\equiv CH$ |
| 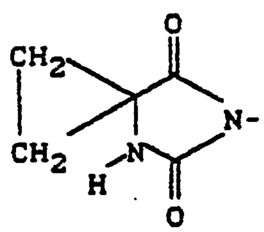 | F | $CH_3$ | $-CH_2-CH=CH_2$ |

| Het | $R^1$ | $R^2$ | $R^3$ |
|---|---|---|---|
| 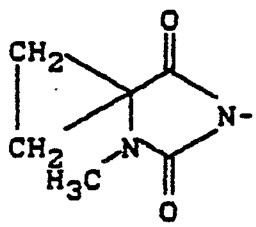 | F | $CH_3$ | $-CH(CH_3)_2$ |
| 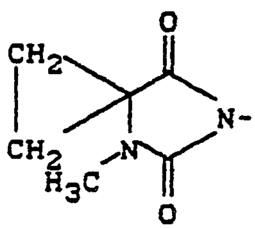 | F | $CH_3$ | $-CH_2-C{\equiv}CH$ |
| 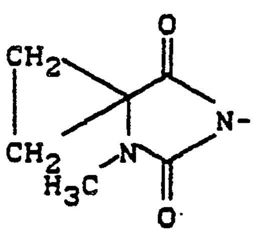 | F | $CH_3$ | $-CH_2-CH{=}CH_2$ |
| 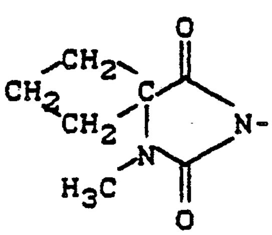 | F | $CH_3$ | $-CH(CH_3)_2$ |
| 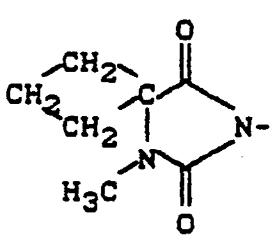 | F | $CH_3$ | $-CH_2-CH(CH_3)_2$ |

| Het | $R^1$ | $R^2$ | $R^3$ |
|---|---|---|---|
| 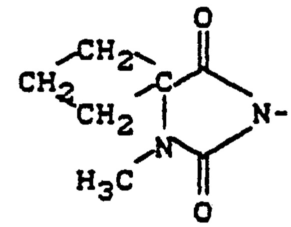 | F | $CH_3$ | $-CH_2-C\equiv CH$ |
| 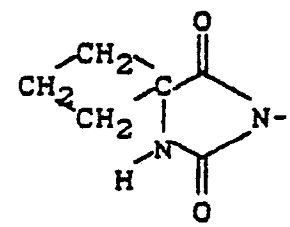 | F | $CH_3$ | $-CH(CH_3)_2$ |
| 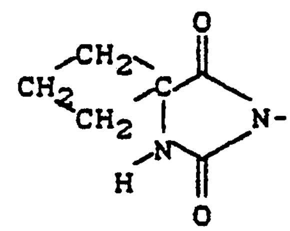 | F | $CH_3$ | $-CH_2-C\equiv CH$ |
| 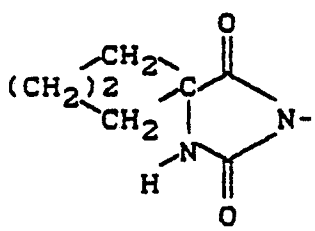 | F | $CH_3$ | $-CH(CH_3)_2$ |
| 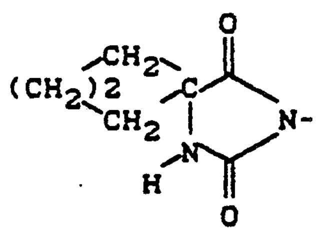 | F | $CH_3$ | $-CH_2-C\equiv CH$ |

| Het | $R^1$ | $R^2$ | $R^3$ |
|---|---|---|---|
| 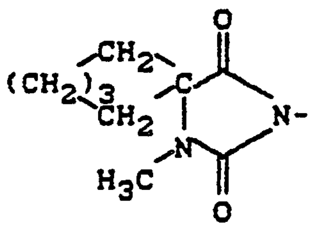 | F | $CH_3$ | $-CH(CH_3)_2$ |
| 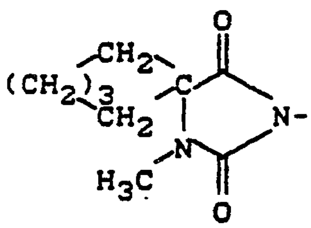 | F | $CH_3$ | $-CH_2-C\equiv CH$ |
| 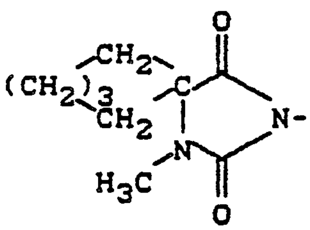 | F | $CH_3$ | $-CH_2-CH=CH_2$ |
| 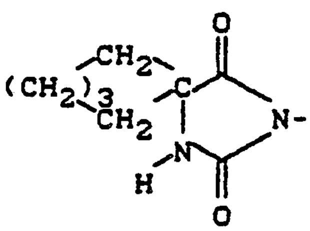 | F | $CH_3$ | $-CH(CH_3)_2$ |
| 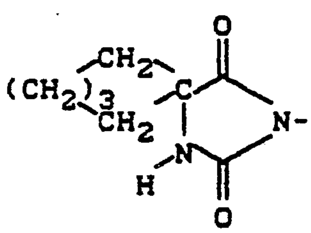 | F | $CH_3$ | $-CH_2-C\equiv CH$ |

| Het | $R^1$ | $R^2$ | $R^3$ |
|---|---|---|---|
| 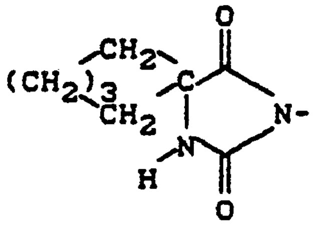 | F | $CH_3$ | $-CH_2-CH=CH_2$ |
| 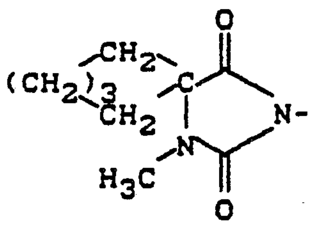 | F | $CH_3$ | $-CH(CH_3)_2$ |
| 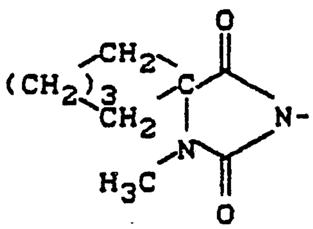 | F | $CH_3$ | $-CH_2-CH(CH_3)_2$ |
| 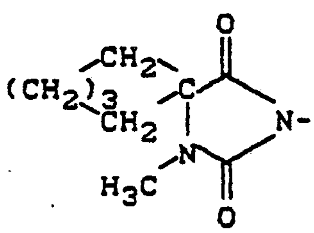 | F | $CH_3$ | $-CH_2-C{\equiv}CH$ |
| 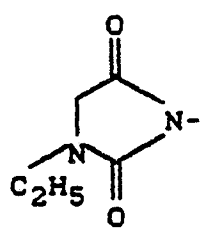 | F | $CH_3$ | $-CH(CH_3)_2$ |

| Het | $R^1$ | $R^2$ | $R^3$ |
|---|---|---|---|
| 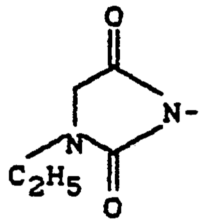 | F | $CH_3$ | $-CH_2-C{\equiv}CH$ |
| 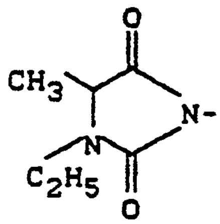 | F | $CH_3$ | $-CH(CH_3)_2$ |
| 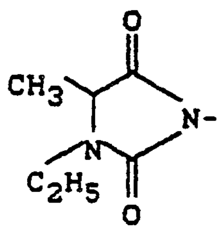 | F | $CH_3$ | $-CH_2-C{\equiv}CH$ |
| 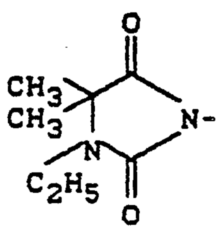 | F | $CH_3$ | $-CH(CH_3)_2$ |
| 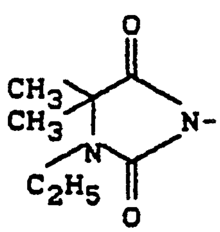 | F | $CH_3$ | $-CH_2-C{\equiv}CH$ |

| Het | $R^1$ | $R^2$ | $R^3$ |
|---|---|---|---|
| 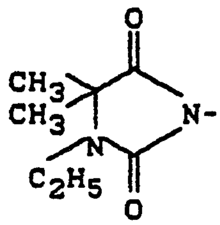 | F | $CH_3$ | $-CH_2-CH=CH_2$ |
| 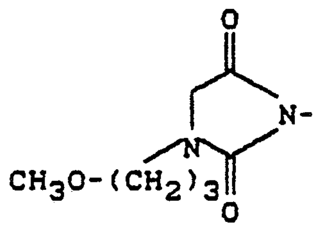 | F | $CH_3$ | $-CH(CH_3)_2$ |
| 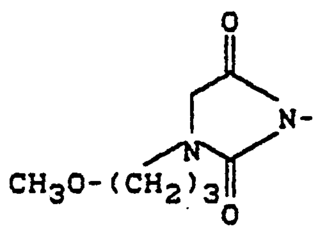 | F | $CH_3$ | $-CH_2-C\equiv CH$ |
| 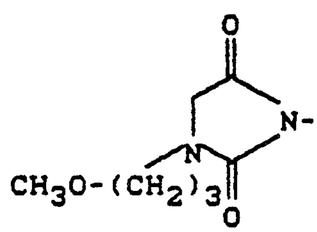 | F | $CH_3$ | $-CH_2-CH=CH_2$ |
| 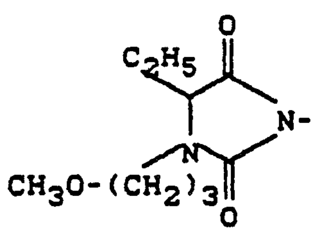 | F | $CH_3$ | $-CH(CH_3)_2$ |

| Het | $R^1$ | $R^2$ | $R^3$ |
|---|---|---|---|
| 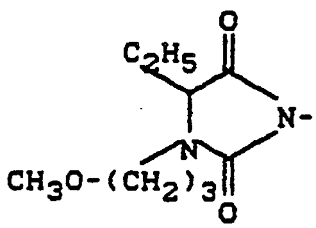 | F | $CH_3$ | $-CH_2-CH(CH_3)_2$ |
| 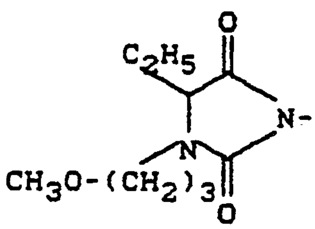 | F | $CH_3$ | $-CH_2-C\equiv CH$ |
| 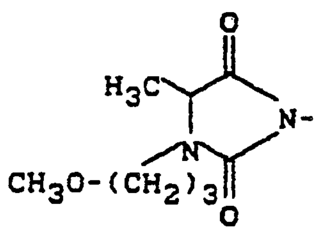 | F | $CH_3$ | $-CH_2-C\equiv CH$ |
| 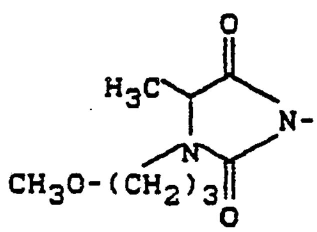 | F | $CH_3$ | $-CH_2-C\equiv CH$ |
| 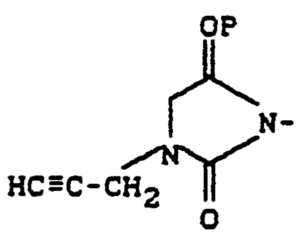 | F | $CH_3$ | $-CH(CH_3)_2$ |

| Het | $R^1$ | $R^2$ | $R^3$ |
|---|---|---|---|
| 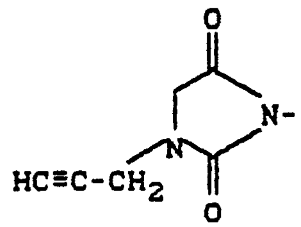 | F | $CH_3$ | $-CH_2-C{\equiv}CH$ |
| 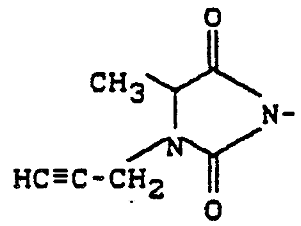 | F | $CH_3$ | $-CH(CH_3)_2$ |
| 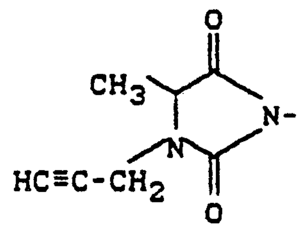 | F | $CH_3$ | $-CH_2-C{\equiv}CH$ |
| 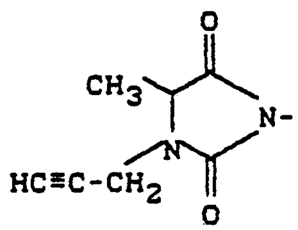 | F | $CH_3$ | $-CH_2-CH{=}CH_2$ |
| 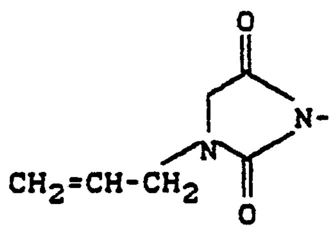 | F | $CH_3$ | $-CH(CH_3)_2$ |

| Het | $R^1$ | $R^2$ | $R^3$ |
|---|---|---|---|
| 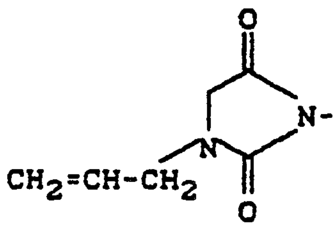 | F | $CH_3$ | $-CH_2-C\equiv CH$ |
| 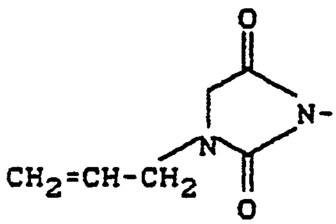 | F | $CH_3$ | $-CH_2-CH=CH_2$ |
| 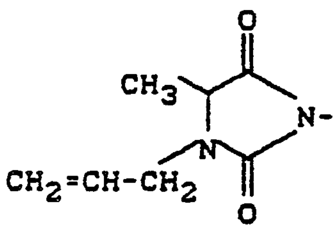 | F | $CH_3$ | $-CH(CH_3)_2$ |
| 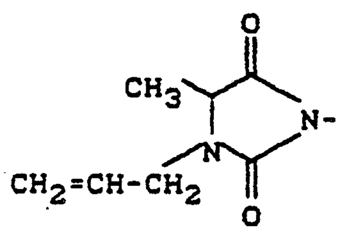 | F | $CH_3$ | $-CH_2-CH(CH_3)_2$ |
| 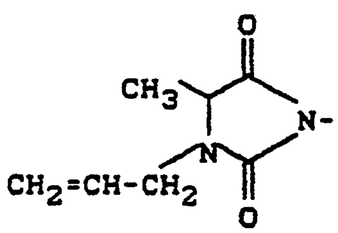 | F | $CH_3$ | $-CH_2-C\equiv CH$ |

| Het | $R^1$ | $R^2$ | $R^3$ |
|---|---|---|---|
| 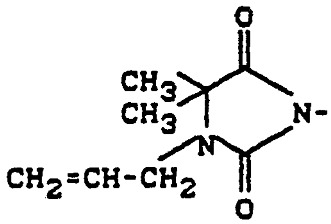 | F | $CH_3$ | $-CH(CH_3)_2$ |
| 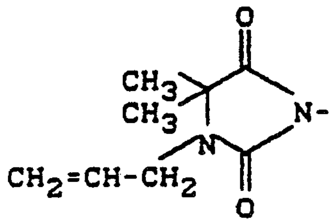 | F | $CH_3$ | $-CH_2-C\equiv CH$ |
| 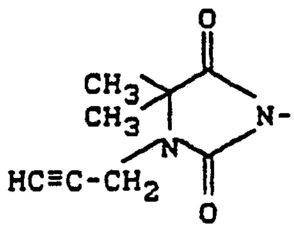 | F | $CH_3$ | $-CH(CH_3)_2$ |
| 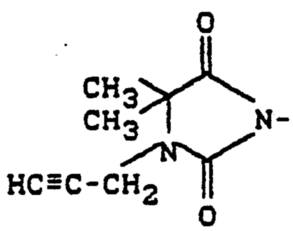 | F | $CH_3$ | $-CH_2-C\equiv CH$ |
| 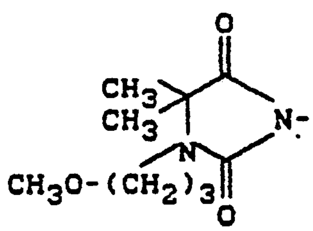 | F | $CH_3$ | $-CH(CH_3)_2$ |

| Het | $R^1$ | $R^2$ | $R^3$ |
|---|---|---|---|
| 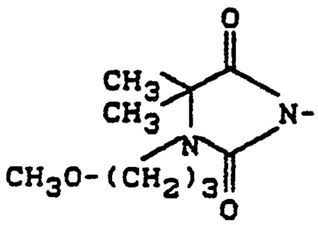 | F | $CH_3$ | $-CH_2-C{\equiv}CH$ |
| 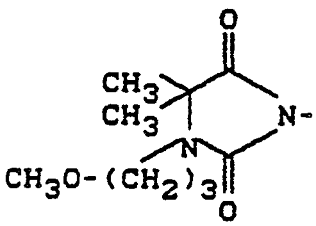 | F | $CH_3$ | $-CH_2-CH{=}CH_2$ |
| 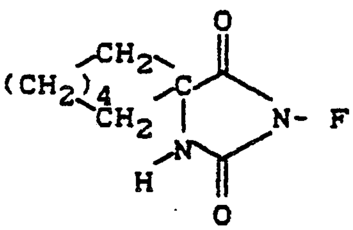 | F | $CH_3$ | $-CH(CH_3)_2$ |
| 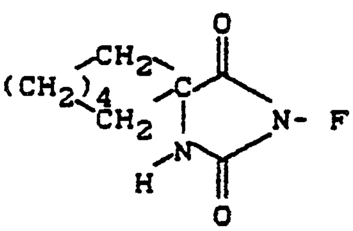 | F | $CH_3$ | $-CH_2-C{\equiv}CH$ |
| 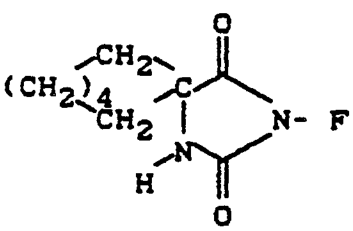 | F | $CH_3$ | $-CH_2-CH{=}CH_2$ |

| Het | $R^1$ | $R^2$ | $R^3$ |
|---|---|---|---|
| 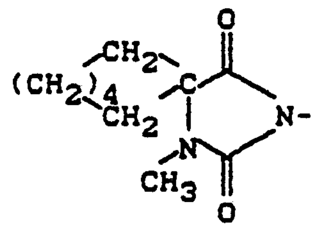 | F | $CH_3$ | $-CH(CH_3)_2$ |
| 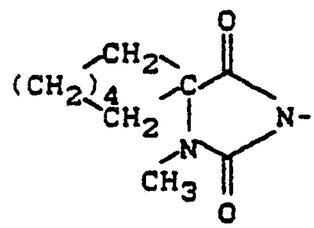 | F | $CH_3$ | $-CH_2-CH(CH_3)_2$ |
| 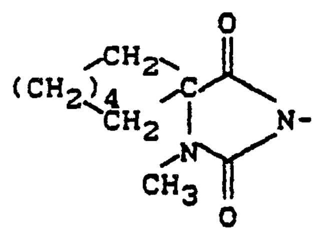 | F | $CH_3$ | $-CH_2-C{\equiv}CH$ |
| 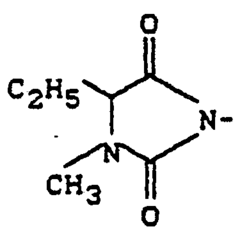 | F | $CH_3$ | $-CH(CH_3)_2$ |
| 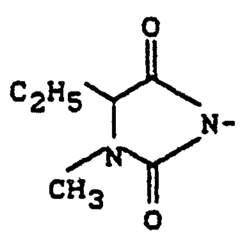 | F | $CH_3$ | $-CH_2-C{\equiv}CH$ |

Verwendet man beispielsweise 2-(2-Fluor-5-hydroxy-4-methylphenyl)-hexahydroimidazo[1,5-a]-pyridin-1,3-dion und Allylbromid als Ausgangsstoffe, so läßt sich der Reaktionsablauf des erfindungsgemäßen Verfahrens (a-α) durch das folgende Formelschema darstellen:

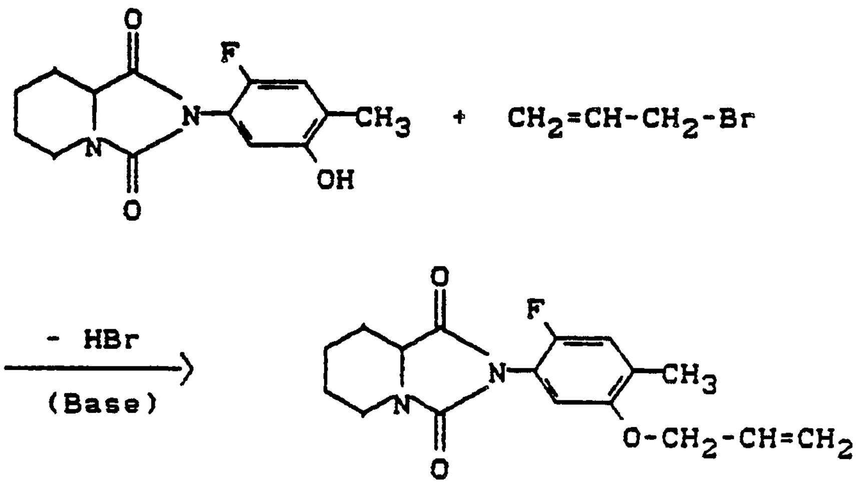


Verwendet man beispielsweise 2-(3-Hydroxy-4-methylphenyl)-tetrahydroimidazol[5,1-c]-[1,4]-thiazin-1,3-(2H)-dion und Propionylchlorid als Ausgangsstoffe, so läßt sich der Reaktionsablauf des erfindungsgemäßen Verfahrens (a-ß) durch das folgende Formelschema darstellen:

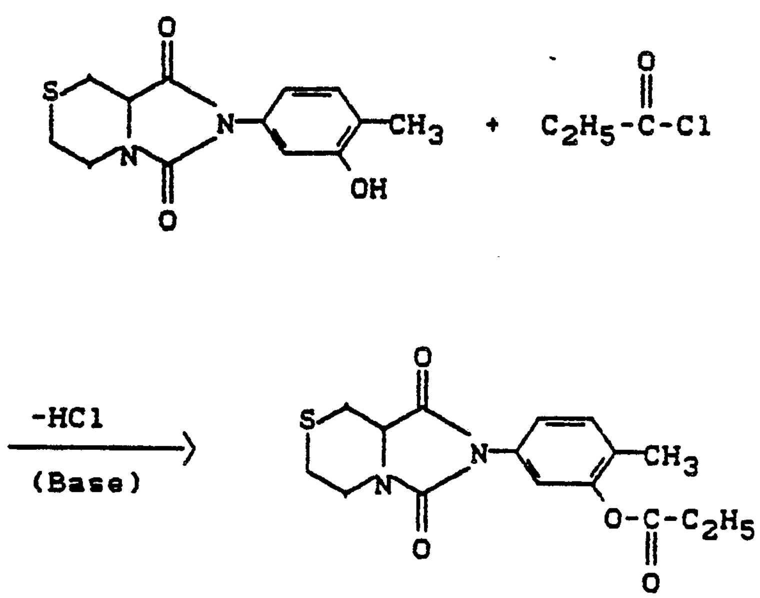


Verwendet man beispielsweise 6-(2-Fluor-5-hydroxy-4-methylphenyl)-1H,3H-imidazo[1,5-c]thiazol-5,7-[6H,7aH)-dion und Trifluormethansulfonsäurechlorid als Ausgangsstoffe, so läßt sich der Reaktionsablauf des erfindungsgemäßen Verfahrens (a-γ) durch das folgende Formelschema darstellen:

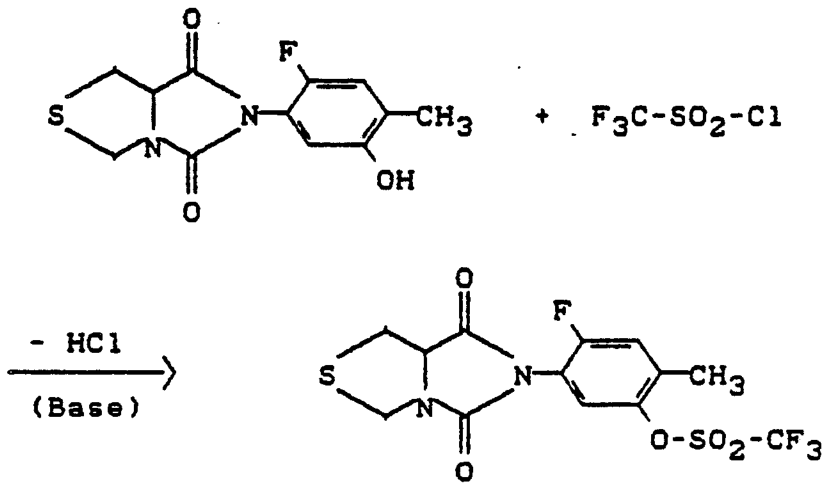


Verwendet man beispielsweise 2-Piperidinylcarbonsäureethylester und (2-Fluor-5-isopropoxy-4-methylphenyl)-isothiocyanat als Ausgangsstoffe, so läßt sich der Reaktionsablauf des erfindungsgemäßen Verfahrens (b-α) durch das folgende Formelschema darstellen:

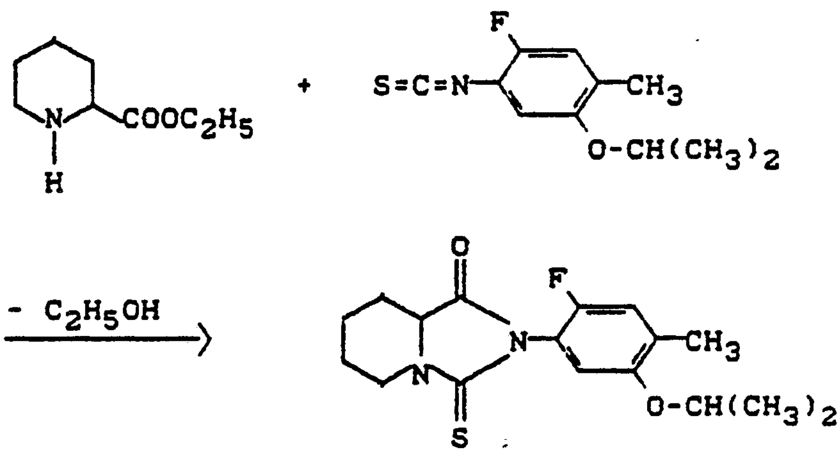


Verwendet man beispielsweise Thiazolidin-4-carbonsäureethylester Hydrochlorid und N-(3-Propargyloxy-4-methylphenyl)-O-phenylcarbamat als Ausgangsstoffe, so läßt sich der Reaktionsablauf des erfindungsgemäßen Verfahrens (b-ß) durch das folgende Formelschema darstellen:

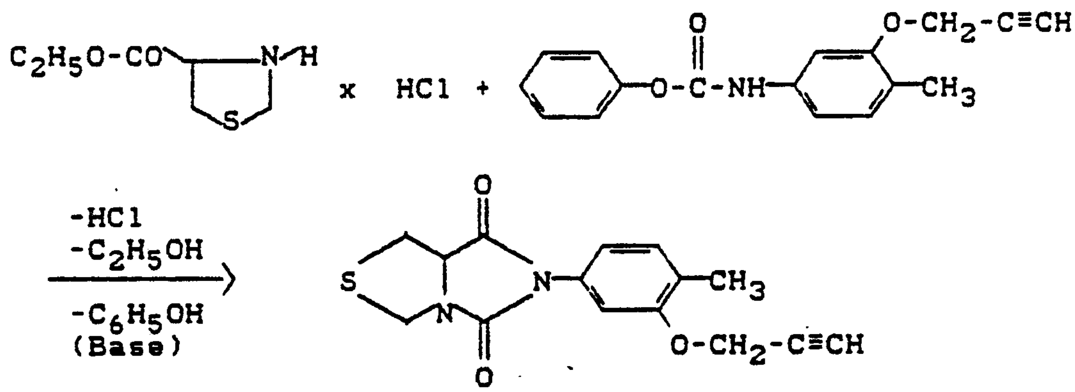


Verwendet man beispielsweise 2-(5-Allyloxy-2-fluor-4-methylphenyl)-hexahydroimidazo[1,5-a]pyridin-1,3-dion und "Lawessons Reagenz" als Ausgangsstoffe, so läßt sich der Reaktionsablauf des erfindungsgemäßen Verfahrens (c) durch das folgende Formelschema darstellen:

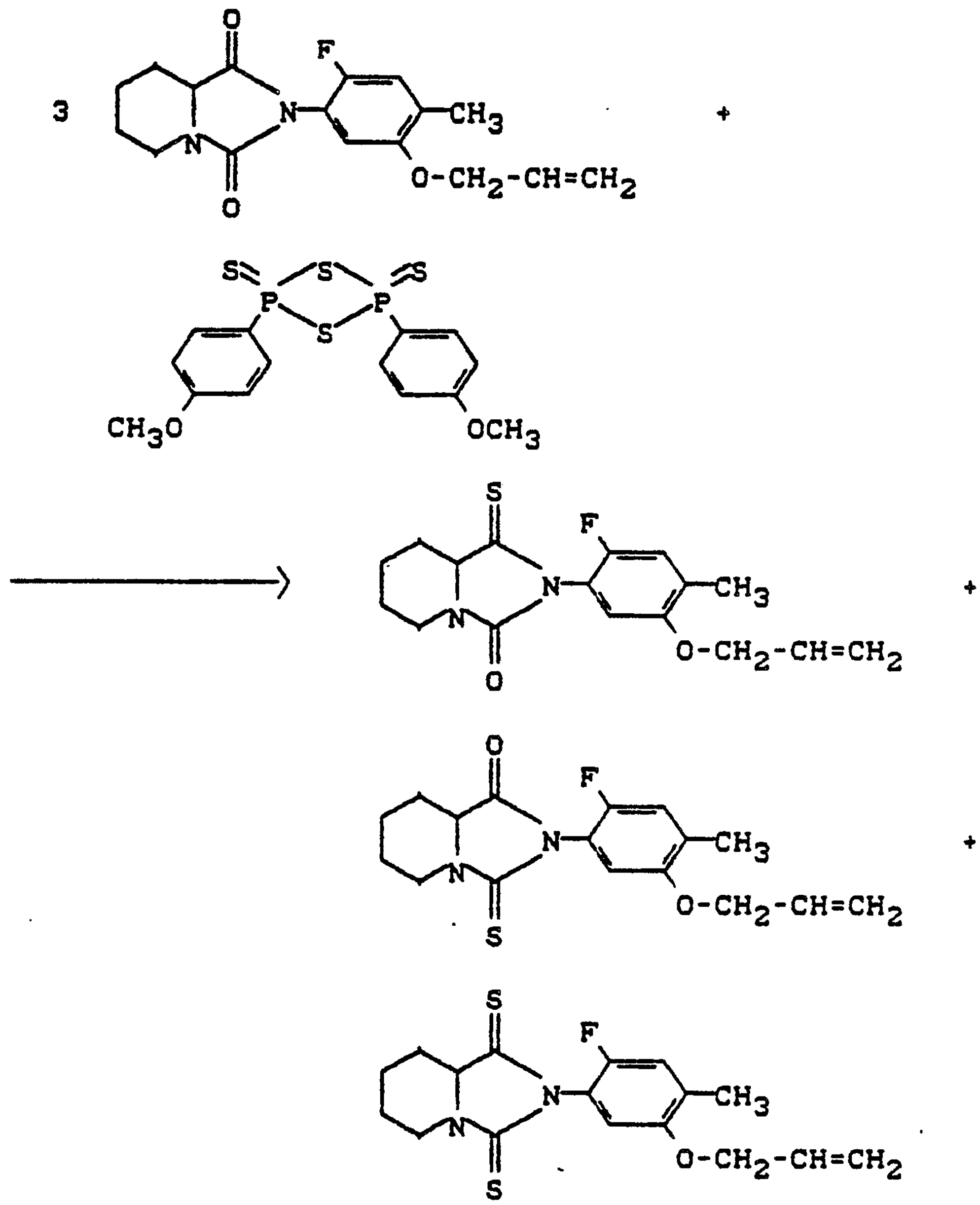

Die zur Durchführung des erfindungsgemäßen Verfahrens (a) als Ausgangsstoffe benötigten Phenolderivate sind durch die Formel (II) allgemein definiert. In dieser Formel (II) stehen $R^1$, $R^2$ und Het vorzugsweise für diejenigen Reste, die bereits im Zusammenhang mit der Beschreibung der erfindungsgemäßen Stoffe der Formel (I) als bevorzugt für diese Substituenten genannt wurden.

Die Phenolderivate der Formel (II) sind noch nicht bekannt. Man erhält sie, wenn man Aniline der Formel (VIII),

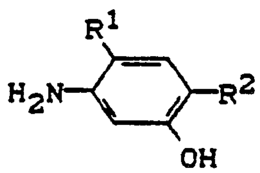 (VIII)

in welcher
$R^1$ und $R^2$ die oben angegebene Bedeutung haben,
zunächst mit Chlorameisensäurephenylester oder (Thio)Phosgen gegebenenfalls in Gegenwart eines Verdünnungsmittels wie beispielsweise Dichlormethan und gegebenenfalls in Gegenwart eines Reaktionshilfsmittels wie beispielsweise Natriumhydroxid oder Triethylamin bei Temperaturen zwischen - 20 °C und + 80 °C umsetzt und anschließend die so erhältlichen Carbamate der Formel (IX),

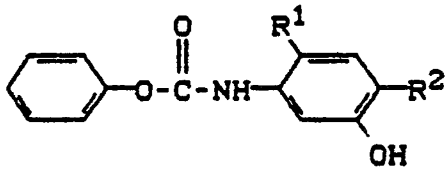
(IX)

in welcher
$R^1$ und $R^2$ die oben angegebene Bedeutung haben,
oder Iso(thio)cyanate der Formel (X),

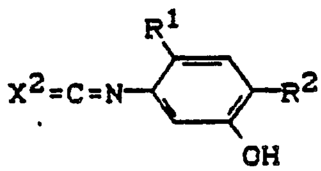
(X)

in welcher
$X^2$ für Sauerstoff oder Schwefel steht und
$R^1$ und $R^2$ die oben angegebene Bedeutung haben,
mit Carbonsäureestern der Formel (IV),
$R^9$-COO$R^{10}$ (IV)
in welcher
$R^9$ für einen Rest der Formel

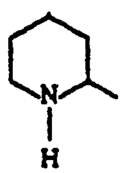 ; 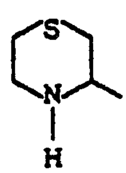 ; 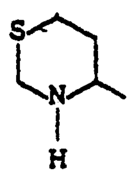 ; 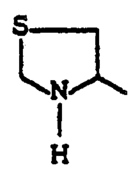

oder

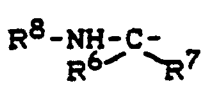

steht,
wobei $R^6$, $R^7$ und $R^8$ die oben angegebene Bedeutung haben und
$R^{10}$ für Alkyl, insbesondere für Methyl oder Ethyl steht,
oder mit deren Säureadditionssalzen gegebenenfalls in Gegenwart eines Verdünnungsmittels wie beispielsweise n-Propanol oder Toluol und gegebenenfalls in Gegenwart eines Reaktionshilfsmittels wie beispielsweise Triethylamin bei Temperaturen zwischen 20 °C und 120 °C in Analogie zur Durchführung des erfindungsgemäßen Verfahrens (b-α) bzw. (b-ß) umsetzt und gegebenenfalls anschließend die so erhältlichen Phenolderivate der Formel (IIa),

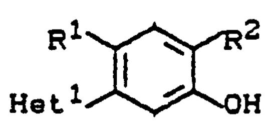
(IIa)

in welcher

$Het^1$ für einen Heterocyclus der Formel

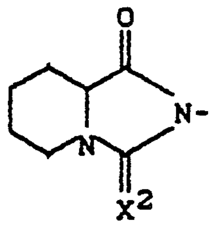 ; 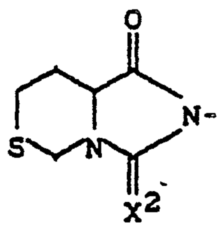 ; 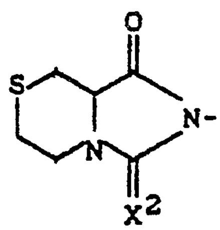 ;

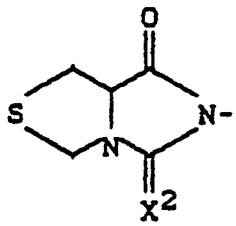 oder 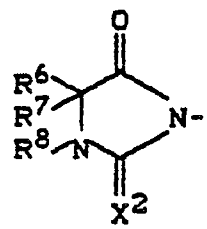 steht,

wobei
$R^1$, $R^2$, $R^6$, $R^7$, $R^8$ und $X^2$ die oben angegebene Bedeutung haben,
Mit "Lawessons Reagenz" der Formel (VII),

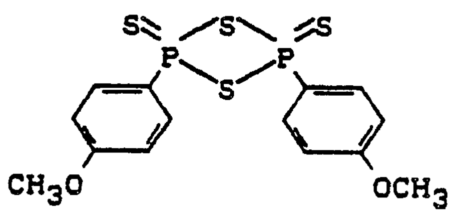

(VII)

gegebenenfalls in Gegenwart eines Verdünnungsmittels wie beispielsweise Toluol bei Temperaturen zwischen + 20 °C und + 150 °C in Analogie zur Durchführung des erfindungsgemäßen Verfahrens (c) umsetzt.

Aniline der Formel (VIII) erhält man, wenn man Nitrophenole der Formel (XI),

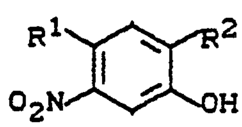

(XI)

in welcher
$R^1$ und $R^2$ die oben angegebene Bedeutung haben,
mit üblichen Reduktionsmitteln, wie beispielsweise molekularem Wasserstoff in Gegenwart eines geeigneten Katalysators wie beispielsweise Raney-Nickel oder Platinoxid gegebenenfalls in Gegenwart eines Verdünnungsmittels wie beispielsweise Tetrahydrofuran oder Ethanol bei Temperaturen zwischen 0 °C und 80 °C und einem Druck zwischen 1 und 50 bar in allgemein üblicher Art und Weise reduziert.

Nitrophenole der Formel (XI) sind teilsweise bekannt (vgl. z.B. GB 1 100 219).
Noch nicht bekannt sind Nitrophenole der Formel (XIa),

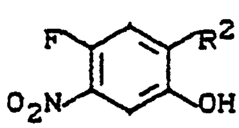

(XIa)

in welcher

$R^2$ die oben angegebene Bedeutung hat.

Man erhält sie, wenn man Fluorphenole der Formel (XII),

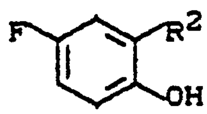

(XII)

in welcher
$R^2$ die oben angegebene Bedeutung hat,
zunächst in allgemein bekannter Weise mit Chlorameisensäuremethylester gegebenenfalls in Gegenwart einer Base wie beispielsweise Natriumhydroxid an der phenolischen OH-Gruppe acyliert, dann in einer 2. Stufe die so erhältlichen Phenylcarbonate der Formel (XIII),

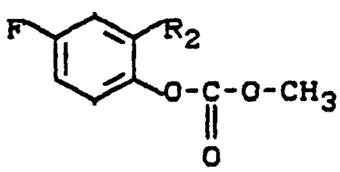

(XIII)

in welcher
$R^2$ die oben angegebene Bedeutung hat,
mit Salpetersäure gegebenenfalls in Gegenwart eines Katalysators wie beispielsweise Schwefelsäure bei Temperaturen zwischen 0 °C und 50 °C nitriert und schließlich in einer 3. Stufe die Nitrophenylcarbonate der Formel (XIV),

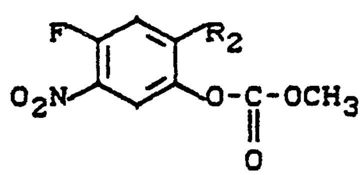

(XIV)

in welcher
$R^2$ die oben angegebene Bedeutung hat,
mit Salzsäure gegebenenfalls in Gegenwart eines Verdünnungsmittels wie beispielsweise Dioxan bei Temperaturen zwischen 50 °C und 120 °C deacyliert.

Fluorierte Phenole der Formel (XII) sind bekannt (vgl. z.B. EP 18 606; Chem. Ber. 86, 501 - 507 [1953]; J. Amer. chem. Soc. 61, 161 - 165 [1939]).

Die zur Durchführung des erfindungsgemäßen Verfahrens (a-α) weiterhin als Ausgangsstoffe benötigten Alkylierungsmittel sind durch die Formel (IIIa) allgemein definiert. In dieser Formel (IIIa) steht $R^{3-1}$ vorzugsweise für diejenigen Reste, die bereits im Zusammenhang mit der Beschreibung der erfindungsgemäßen Stoffe der Formel (I) als bevorzugt für den Substituenten $R^3$ genannt wurden, mit Ausnahme der Reste $-\underset{\underset{O}{\|}}{C}-R^4$
sowie $-SO_2-R^5$.

$R^{3-1}$ steht besonders bevorzugt für Methyl, Ethyl, n-oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom, für jeweils geradkettiges oder verzweigtes Cyanalkyl, Alkoxyalkyl, Alkoxyalkoxyalkyl oder Alkoxycarbonylalkyl mit jeweils 1 bis 3 Kohlenstoffatomen in den einzelnen Alkylteilen, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl, Cyclohexyl, Oxetanylmethyl, Tetrahydrofuranylmethyl oder Tetrahydropyranylmethyl oder für

jeweils gegebenenfalls ein-bis dreifach gleich oder verschieden substituiertes Benzyl oder Phenylethyl, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio.
$E^1$ steht vorzugsweise für Halogen, insbesondere für Brom oder Iod, für Alkoxysulfonyloxy oder für gegebenenfalls substituiertes Arylsulfonyloxy wie beispielsweise Methoxysulfonyloxy, Ethoxysulfonyloxy oder p-Toluolsulfonyloxy.

Die Alkylierungsmittel der Formel (IIIa) sind allgemein bekannte Verbindungen der organischen Chemie.

Die zur Durchführung des erfindungsgemäßen Verfahrens (a-ß) weiterhin als Ausgangsstoffe benötigten Acylierungsmittel sind durch die Formel (IIIb) allgemein definiert. In dieser Formel (IIIb) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.
$E^2$ steht vorzugsweise für Halogen, insbesondere für Chlor oder Brom oder für einen Rest $-O-\underset{O}{\underset{\|}{C}}-R^4$,
wobei $R^4$ die oben angegebene Bedeutung hat.

Die Acylierungsmittel der Formel (IIIb) sind allgemein bekannte Verbindungen der organischen Chemie.

Die zur Durchführung des erfindungsgemäßen Verfahrens (a-γ) weiterhin als Ausgangsstoffe benötigten Sulfonylierungsmittel sind durch die Formel (IIIc) allgemein definiert. In dieser Formel (IIIc) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.
$E^3$ steht vorzugsweise für Halogen, insbesondere für Fluor, Chlor oder Brom.

Die Sulfonylierungsmittel der Formel (IIIc) sind ebenfalls allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten Carbonsäureester sind durch die Formel (IV) allgemein definiert. In dieser Formel (IV) steht
$R^9$ vorzugsweise für geradkettiges oder verzweigtes Alkyl mit 1 bis 4 Kohlenstoffatomen, insbesondere für Methyl oder Ethyl.
$R^{10}$ steht vorzugsweise für einen Rest der Formel

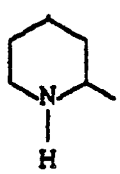 ; 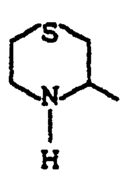 ; 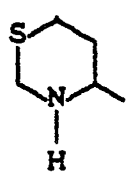 ; 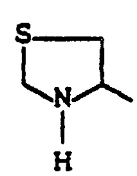

oder

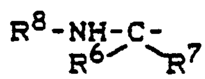 ;

insbesondere für einen Rest der Formel

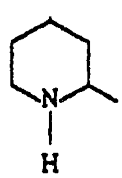 ; 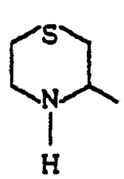 ; 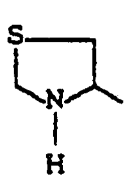 oder 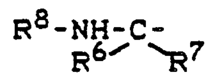 ,

wobei
$R^6$, $R^7$ und $R^8$ vorzugsweise für diejenigen Reste stehen, die bereits im Zusammenhang mit der Beschreibung der erfindungsgemäßen Verbindungen der Formel (I) bevorzugt für diese Substituenten genannt wurden.

Die Carbonsäureester der Formel (IV) sowie deren Säureadditonssalze, wie insbesondere deren

Hydrochloride, sind allgemein bekannt oder erhältlich in Analogie zu bekannten Verfahren (vgl. z.B. Tetrahedron 29, 3389 [1973]; DE-OS 23 31 549; J. chem. Soc. Chem. Commun. 1972, 589-590; J. Med. Chem. 14, 501-508 [1971]; J. Heterocycl. Chem. 6, 181-185 (1969)].

Die zur Durchführung des erfindungsgemäßen Verfahrens (b-α) weiterhin als Ausgangsstoffe benötigten Iso(thio)cyanate sind durch die Formel (V) allgemein definiert. In dieser Formel (V) stehen $R^1$, $R^2$, $R^3$ und $X^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (V) sind teilweise bekannt (vgl. z.B. US-PS 40 13 452; Justus Liebigs Ann. Chem. 1976, 1689-1712; US-PS 39 37 726 und DE-OS 23 34 355).

Noch nicht bekannt sind Iso(thio)cyanate der Formel (Va),

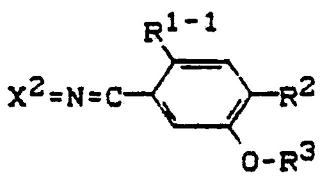

(Va)

in welcher
$R^{1-1}$ für Halogen steht und
$R^2$, $R^3$ und $X^2$ die oben angegebene Bedeutung haben.

Man erhält sie, wenn man Aniline der Formel (XVa),

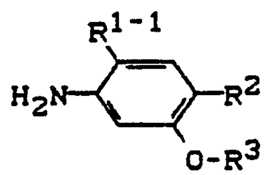

(XVa)

in welcher
$R^{1-1}$, $R^2$ und $R^3$ die oben angegebene Bedeutung haben,
mit Phosgen oder Thiophosgen, gegebenenfalls in Gegenwart eines Verdünnungsmittels wie beispielsweise Chloroform oder Toluol und gegebenenfalls in Gegenwart eines Reaktionshilfsmittels wie beispielsweise Chlorwasserstoffgas oder Triethylamin bei Temperaturen zwischen - 20 °C und + 150 °C umsetzt (vgl. auch die Herstellungsbeispiele).

Die zur Durchführung des erfindungsgemäßen Verfahrens (b-ß) weiterhin als Ausgangsstoffe benötigten Carbamate sind durch die Formel (VI) allgemein definiert. In dieser Formel (VI) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Carbamate der Formel (VI) sind noch nicht bekannt. Man erhält sie, wenn man Aniline der Formel (XV),

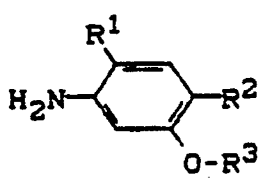

(XV)

in welcher
$R^1$, $R^2$ und $R^3$ die oben angegebene Bedeutung haben,
mit Chlorameisensäurephenylester gegebenenfalls in Gegenwart eines Verdünnungsmittels wie beispielsweise Chloroform und gegebenenfalls in Gegenwart eines Reaktionshilfsmittels wie beispielsweise Triethylamin bei Temperaturen zwischen - 20 °C und + 150 °C umsetzt.

Aniline der Formel (XV) bzw. (XVa) sind teilweise bekannt (vgl. z.B. Chem. Ber. 117, 2275 - 2286, [1984]; Bull. chem. Soc. Japan 49, 2294 - 2297, [1976]; Belg. Pat. BE 11 705 vom 06.09.1968 oder J. chem Soc. 1965, 1187 -1191) sowie die eigene vorgängige, nicht vorveröffentlichte Deutsche Patentanmeldung P

37 31 516 vom 18.09.1987) oder können in Analogie zu bekannten Verfahren erhalten werden (vgl. z.B. EP 165 895 oder Roczniki Chem. 38, 51 - 59 [1964] bzw. CA 60 : 14 434 f).

Noch nicht bekannt sind Aniline der Formel (XVb),

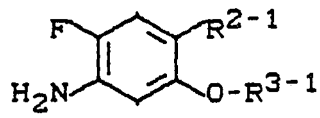

(XVb)

in welcher
$R^{2-1}$ für Alkyl steht und
$R^{3-1}$ für -CO-$R^4$ steht, wobei
$R^{4-1}$ für Alkoxyalkyl oder Alkoxyalkylamino steht.

Bevorzugt sind die Aniline der Formel (XVb), in welcher
$R^{2-1}$ für Methyl, Ethyl, n- oder iso-Propyl, n-, iso, sec.- oder tert.-Butyl steht,
$R^{3-1}$ für -CO-$R^4$ steht, wobei
$R^{4-1}$ für jeweils geradkettiges oder verzweigtes Alkoxyalkyl oder Alkoxyalkylamino mit jeweils 1 bis 6 Kohlenstoffatomen in den einzelnen Alkyl- bzw. Alkoxyteilen steht.

Besonders bevorzugt sind diejenigen Aniline der Formel (XVb), in welcher
$R^{2-1}$ für Methyl, Ethyl, n- oder iso-Propyl, n-, iso-, sec.- oder tert.-Butyl, insbesondere für Methyl oder Ethyl steht und
$R^{3-1}$ für -CO-$R^4$ steht, wobei
$R^{4-1}$ für jeweils geradkettiges oder verzweigtes Alkoxyalkyl oder Alkoxyalkylamino mit jeweils 1 bis 3 Kohlenstoffatomen in den einzelnen Alkyl- bzw. Alkoxyteilen steht.

Man erhält neue und bekannte Aniline der Formel (XV), beispielsweise, wenn man fluorierte Phenolether der Formel (XVI),

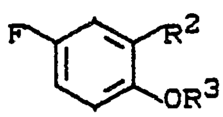

(XVI)

in welcher
$R^2$ und $R^3$ die oben angegebene Bedeutung haben,
zunächst in einer 1. Stufe mit einem üblichen Nitrierungsmittel, wie beispielsweise Salpetersäure gegebenenfalls in Gegenwart eines Katalysators wie beispielsweise Schwefelsäure bei Temperaturen zwischen - 20 °C und + 30 °C nitriert, und die so erhältlichen Nitroverbindungen der Formel (XVII),

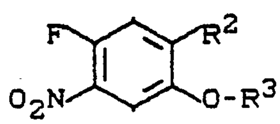

(XVII)

in welcher
$R^2$ und $R^3$ die oben angegebene Bedeutung haben,
mit üblichen Reduktionsmitteln wie beispielsweise molekularem Wasserstoff in Gegenwart eines Katalysators wie beispielsweise Raney-Nickel gegebenenfalls in Gegenwart eines Verdünnungsmittels wie beispielsweise Tetrahydrofuran bei Temperaturen zwischen 0 °C und 80 °C und einem Druck zwischen 1 und 60 bar in allgemein üblicher Art und Weise reduziert.

Fluorierte Phenolether der Formel (XVI) sind teilweise bekannt (vgl. z.B. Liebigs Ann. Chem. 595, 131 - 159 [1955]; J. Amer. chem. Soc. 61, 161 - 165 [1939] oder Bull. acad. Polon. Sci.; Sci. Chim. 11, 117 - 120 [1963] bzw. CA 59: 7408a) oder erhältlich in Analogie zu allgemein bekannten Verfahren, beispielsweise, indem man fluorierte Phenole der Formel (XII),

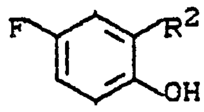

(XII)

in welcher

$R^2$ die oben angegebene Bedeutung hat,

in allgemein üblicher Art und Weise alkyliert, acyliert oder sulfonyliert (vgl. hierzu die Angaben zu den erfindungsgemäßen Verfahren (a-α), (a-ß) und (a-γ) sowie die Herstellungsbeispiele).

Alternativ erhält man Aniline der Formel (XV) auch, wenn man Aniline der Formel (VIII),

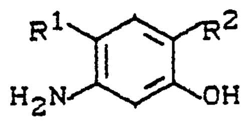

(VIII)

in welcher

$R^1$ und $R^2$ die oben angegebene Bedeutung haben,

zunächst in allgemein bekannter Art und Weise mit üblichen Aminoschutzgruppereagenzien wie beispielsweise Acetylchlorid in Gegenwart von Triethylamin an der Aminogruppe schützt (vgl. z.B. Ber. dtsch. chem. Ges. 17, 609 [1884]), anschließend ebenfalls in allgemein bekannter Art und Weise an der phenolischen Hydroxygruppe alkyliert, acyliert oder sulfonyliert (vgl. hierzu die Angaben zu den erfindungsgemäßen Verfahren (a-α), (a-ß) und (a-γ) sowie die Herstellungsbeispiele) und schließlich die Aminoschutzgruppe ebenfalls in allgemein üblicher Art und Weise, beispielsweise mit Chlorwasserstoff in Methanol wieder abspaltet (vgl. auch die Herstellungsbeispiele).

Die zur Durchführung des erfindungsgemäßen Verfahrens (c) als Ausgangsstoffe benötigten N-Arylstickstoffheterocyclen sind durch die Formel (Ia) allgemein definiert. In dieser Formel (Ia) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Het[1] steht vorzugsweise für einen Heterocyclus der Formel

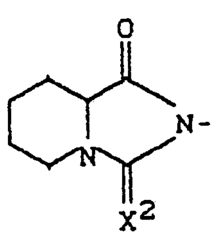 ; 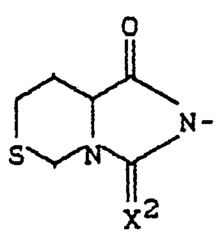 ; 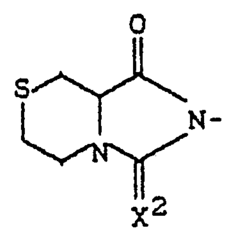 ;

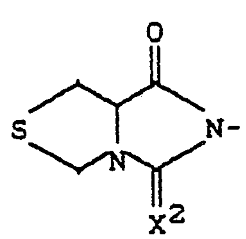 oder 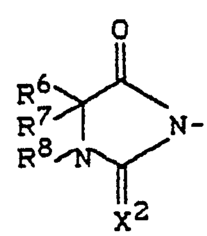 ,

wobei $X^2$ jeweils für Sauerstoff oder Schwefel steht und $R^6$, $R^7$ und $R^8$ vorzugsweise für diejenigen Reste stehen, die bereits im Zusammenhang mit der Beschreibung der erfindungsgemäßen Stoffe der Formel (I) als bevorzugt für diese Substituenten genannt wurden.

Die N-Arylstickstoffheterocyclen der Formel (Ia) sind erfindungsgemäße Verbindungen und erhältlich mit Hilfe der erfindungsgemäßen Verfahren (a) oder (b).

Das zur Durchführung des erfindungsgemäßen Verfahrens (c) weiterhin als Ausgangsstoff benötigte "Lawesson Reagenz" ist durch die Formel (VII) definiert.

Das "Lawesson Reagenz" der Formel (VII) ist bekannt (vgl. z.B. DE-OS 2 606 083; Bull. Soc. Chim. Belg. 87, 223-228 [1978]).

Zur Durchführung des erfindungsgemäßen Verfahrens (a) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner in den Varianten (a-α), (a-ß) oder (a-γ) Verbindungen der Formeln (IIIa), (IIIb) oder (IIIc) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (a) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (a) setzt man pro Mol an Phenolderivat der Formel (II) in den Varianten (a-α), (a-ß) oder (a-γ) im allgemeinen jeweils 1,0 bis 20,0 Mol, vorzugsweise jeweils 1,0 bis 15,0 Mol, an Alkylierungsmittel der Formel (IIIa) oder Acylierungsmittel der Formel (IIIb) oder Sulfonylierungsmittel der Formel (IIIc) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (I) erfolgt nach üblichen Methoden (vgl. auch die Herstellungsbeispiele).

Als Verdünnungsmittel zur Durchführung des erfindungsgemäßen Verfahren (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole wie Methanol, Ethanol oder Propanol.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (b) setzt man pro Mol an Heterocyclylcarbonsäureester der Formel (IV) oder eines entsprechenden Säureadditionssalzes in den Varianten (b-α) und (b-ß) im allgemeinen 0.5 bis 5.0 Mol, vorzugsweise 0.8 bis 1.5 Mol an Iso(thio)cyanat der Formel (V) oder an Carbamat der Formel (VI) und gegebenenfalls 1.0 bis 10.0 Mol, vorzugsweise 1.0 bis 5.0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und

180 °C, vorzugsweise bei Temperaturen zwischen 20 °C und 150 °C.

Zur Durchführung des erfindungsgemäßen Verfahrens (c) setzt man pro Mol an N-Aryl-Stickstoffheterocyclus der Formel (Ia) im allgemeinen 0.2 bis 2.0 Mol, vorzugsweise 0.5 bis 1.5 Mol an "Lawesson Reagenz" der Formel (VII) ein.

Die Reaktionsdurchführung, Aufarbeitung und Isolierung der Reaktionsprodukte erfolgt in Analogie zu bekannten Verfahren (vgl. z.B. Bull. Soc. Chim. Belg. 87, 223-228 [1978]).

In der Regel erhält man bei der Durchführung des erfindungsgemäßen Verfahrens (c) als Endprodukt ein Gemisch aus Verbindungen der Formel (Ib-1),

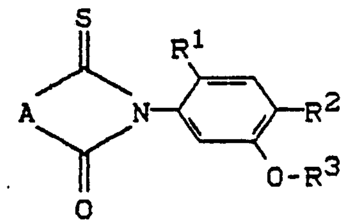

(Ib-1)

und Verbindungen der Formel (Ib-2)

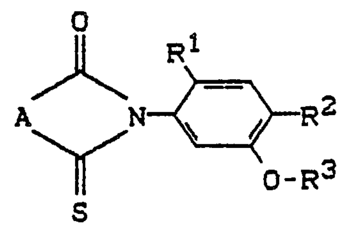

(Ib-2)

und Verbindungen der Formel (Ib-3)

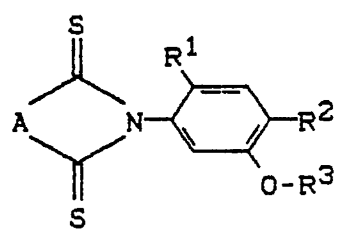

(Ib-3)

wobei

A jeweils für einen Rest der Formel

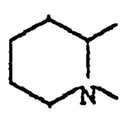 ; 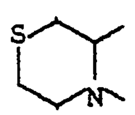 ; 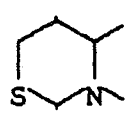 ; 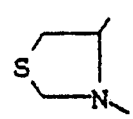 oder

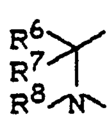 steht und

$R^1$, $R^2$, $R^3$, $R^6$, $R^7$ und $R^8$ die oben angegebene Bedeutung haben.

Diese Gemische lassen sich mit üblichen Trennmethoden (Chromatographie, Kristallisation) auftrennen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen

Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von mono- und dikotylen Unkräutern in mono- und dikotylen Kulturen, wie beispielsweise Soja oder Weisen einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl,

Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on zur (METAMITRON) Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D);
2,4-Dichlorphenoxypropionsäure (2,4-DP);
4-(2,4-Dichlorphenoxy)-buttersäure (2,4-DB);
(2-Methyl-4-chlorphenoxy)-essigsäure (MCPA);
(4-Chlor-2-methylphenoxy)-propionsäure (MCPP);
3,5,6-Trichlor-2-pyridyloxyessigsäure (TRICLOPYR);
2-[4-(2,4-Dichlorphenoxy)-phenoxy]-propionsäure deren Methyl- oder deren Ethylester (DICLOFOP-[METHYL]);
2-{4-[(6-Chlor-2-benzoxazolyl)-oxy]-phenoxy}-propansäure, deren Methyl- oder deren Ethylester (FENOXAPROP);
N-(1-Ethylpropyl)-3,4-dimethyl-2,6-dinitroanilin (PENDIMETHALIN);
Methyl-5-(2,4-dichlorphenoxy)-2-nitrobenzoat (BIFENOX);
N,N-Dimethyl-N′-(3-chlor-4-methylphenyl)-harnstoff (CHLORTOLURON);
N,N-Dimethyl-N′-(4-isopropylphenyl)-harnstoff (ISOPROTURON);
Methyl-2-[4,5-dihydro-4-methyl-4-(1-methylethyl)-5-oxo-1H-imidazol-2-yl]-4(5)-methylbenzoat (IMAZAMETHABENZ);
3,5-Dibrom-4-hydroxy-benzonitril (BROMOXYNIL);
3,5-Diiod-4-hydroxybenzonitril (IOXYNIL);
N-Methyl-2-(1,3-benzthiazol-2-yloxy)-acetanilid (MEFENACET);
Ethyl-2-{[(4-chlor-6-methoxy-2-pyrimidinyl)-aminocarbonyl]-aminosulfonyl}-benzoat (CHLORIMURON);
2-Chlor-N-{[(4-methoxy-6-methyl-1,3,5-triazin-2-yl)-amino]-carbonyl}-benzolsulfonamid (CHLORSULFURON);
2-{[[((4-Methoxy-6-methyl-1,3,5-triazin-2-yl)-amino)-carbonyl]-amino]-sulfonyl}-benzoesäure oder deren Methylester (METSULFURON);
3-[[[[(4-Methoxy-6-methyl-1,3,5-triazin-2-yl)-amino]-carbonyl]-amino]-sulfonyl]-thiophen-2-carbonsäuremethylester (THIAMETHURON);
N,S-Diethyl-N-cyclohexyl-thiolcarbamat (CYCLOATE);
4-Ethylamino-2-t-butylamino-6-methylthio-s-triazin (TERBUTRYNE);
4-Amino-6-t-butyl-3-ethylthio-1,2,4-triazin-5(4H)-on (ETHIOZIN);
3-Isopropyl-2,1,3-benzothiadiazin-4-on-2,2-dioxid (BENTAZON) und 0-(6-Chlor-3-phenyl-pyridazin-4-yl)-S-octyl thiocarbonat (PYRIDATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres

Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können bei der Anwendung als Wachstumregulatoren in den Formulierungen ebenfalls in Mischung mit anderen bekannten Wirkstoffen vorliegen, wie Fungizide, Insektizide, Akarizide und Herbizide sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

## Herstellungsbeispiele

### Beispiel 1

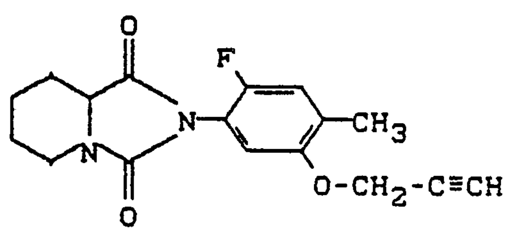

(Verfahren a)

3,93 g (0.015 Mol) 2-(2-Fluor-5-hydroxy-4-methylphenyl)-hexahydroimidazo[1,5-a]pyridin-1,3-dion, 2,77 g (0,020 Mol) Kaliumcarbonat und 1,67 ml (0.018 Mol) Propargylbromid werden in 40 ml absolutem Acetonitril bei 70 °C so lange gerührt bis im Dünnschichtchromatogramm kein Ausgangsprodukt mehr nachweisbar ist (ca. 6 Stunden). Zur Aufarbeitung wird filtriert, das Filtrat im Vakuum eingeengt und der Rückstand aus Essigester/n-Hexan umkristallisiert.

Man erhält 3,4 g (72 % der Theorie) an 2-(2-Fluor-4-methyl-5-propargyloxyphenyl)-hexahydroimidazo-[1,5a]pyridin-1,3-dion vom Schmelzpunkt 142 °C.

### Beispiel 2

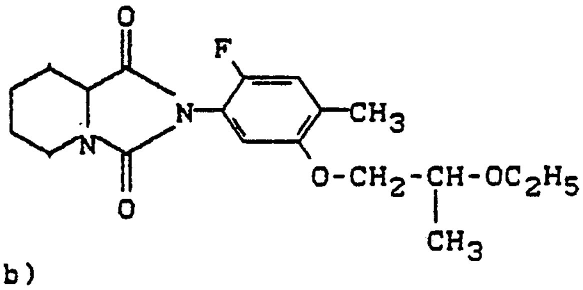

(Verfahren b)

Zu 5 g (0.022 Mol) 5-[(2-Ethoxy)-1-propoxy]-2-fluor-4-methylanilin in 40 ml Dichlormethan gibt man bei 0 °C bis 5 °C tropfenweise unter Rühren gleichzeitig 22 ml (0.022 Mol) 1N-Natronlauge und 3,06 ml (0.024 Mol) Chlorameisensäurephenylester in 10 ml Dichlormethan, rührt nach beendeter Zugabe 15 Minuten nach, trennt dann die Dichlormethanphase ab, trocknet über Natriumsulfat und engt im Vakuum ein. Das so erhältliche Rohprodukt wird in 20 ml n-Propanol aufgenommen, mit 3,76 ml (0.024 Mol) Piperidin-2-carbonsäureethylester versetzt und 8 Stunden unter Rückfluß erhitzt. Zur Aufarbeitung wird im Vakuum eingeengt und der Rückstand an Kieselgel chromatographiert (Laufmittel: Cyclohexan/Essigester 1:1).

Man erhält. 6.2 g (77 % der Theorie) an 2-{5-[(2-Ethoxy)-1-propoxy]-2-fluor-4-methylphenyl}-hexahydroimidazo[1.5-a]pyridin-1,3-dion als Öl.

$^1$H-NMR ($CDCl_3$/Tetramethylsilan):

$\delta$ = 1,18 (d,3H); 1,25 (t, 3H); 2,23 (s,3H); 3,63 (q,2H); 3,75-3,98 (m,3H), 4,25 (m,1H); 6,68 (d,1H), 6,97 (d,1H) ppm.

<u>Beispiel 3</u>

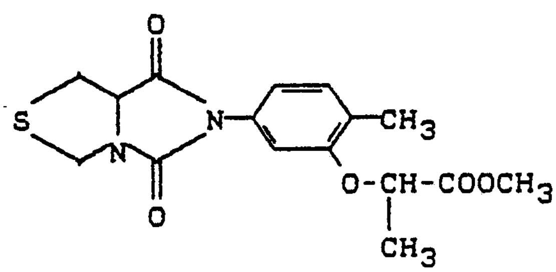

(Verfahren b)

2,37 g (0.012 Mol) Thiazolidin-4-carbonsäureethylester Hydrochlorid (vgl. z.B. Tetrahedron 29, 3389 [1973]), 1,65 g (0.007 Mol) 3-(1-Methoxycarbonylethoxy)-4-methylphenylisocyanat und 2,5 g (0.025 Mol) Triethylamin in 100 ml trockenem Toluol werden 2 Stunden bei Raumtemperatur gerührt, filtriert, das Filtrat mit verdünnter Salzsäure und Wasser gewaschen, über Natriumsulfat getrocknet und im Vakuum eingeengt.

Man erhält 1,7 g (69 % der Theorie) an 6-[3-(1-Methoxycarbonylethoxy)-4-methylphenyl]-1H,3H-imidazo[1,5-c]thiazol-5,7(6H,7aH)-dion als Öl.

$^1$H-NMR ($CDCl_3$/Tetramethylsilan):

$\delta$ = 1,6 (d,3H); 2,3 (s,3H); 3,2 (m,1H); 3,4 (m,1H); 3,8 (s,3H); 4,2 (dd,1H); 4,5 (m,1H); 4,7 (m,1H); 5,1 (d,1H); 6,7 (m,1H), 6,9 (dd,1H); 7,2 (dd,1H) ppm.

<u>Beispiel 4</u>

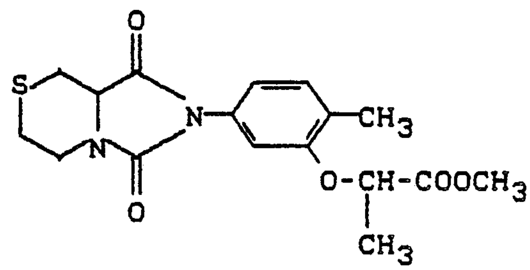

(Verfahren b)

2,1 g (0,012 MOl) 1,4-Thiomorpholin-3-carbonsäureethylester (vgl. z.B. J. Med. Pharm. Chem. 2, 553 [1960]) und 1.65 g (0.007 Mol) 3-(1-Methoxycarbonylethoxy)-4-methyl-phenylisocyanat werden in 100 ml trockenem Toluol 2 Stunden bei Raumtemperatur gerührt, dann mit verdünnter Salzsäure und Wasser gewaschen, über Natriumsulfat getrocknet und im Vakuum eingeengt.

Man erhält 2 g (79 % der Theorie) an 2-[3-(1-Methoxycarbonylethoxy)-4-methylphenyl]-tetrahydroimidazo[5,1-c]-[1,4]thiazin-1,3(2H)-dion als Öl.

$^1$H-NMR ($CDCl_3$/Tetramethylsilan):

$\delta$ = 1,6 (d,3H), 2,3 (s,3H), 2,5-3,5 (m,5H); 3,7 (s,3H); 4,1 (dd,1H); 4,5 (m,1H); 5,4 (m,1H); 6,7 (m,1H); 7,0 (dd,1H); 7,2 (d,1H) ppm.

In entsprechender Weise und gemäß den allgemeinen Angaben zur Herstellung erhält man die folgenden N-Aryl-stickstoffheterocyclen der allgemeinen Formel (I):

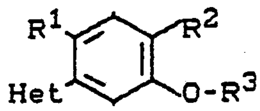

(I)

| Bsp. Nr. | Het | $R^1$ | $R^2$ | $R^3$ | physikalische Eigenschaften |
|---|---|---|---|---|---|
| 5 | 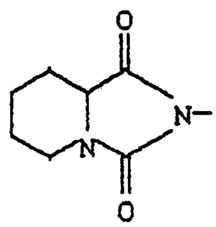 | H | $CH_3$ | $-CH_2-C\equiv CH$ | Fp 138° C |
| 6 | 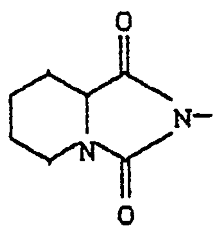 | H | $CH_3$ | $-CH(CH_3)_2$ | $^1$H-NMR*): |
| 7 | 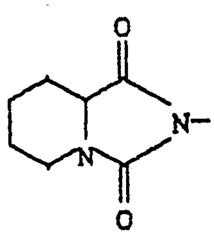 | H | $CH_3$ | $-CH_2-CH=CH_2$ | Fp 89° C |

| Bsp. Nr. | Het | $R^1$ | $R^2$ | $R^3$ | physikalische Eigenschaften |
|---|---|---|---|---|---|
| 8 | 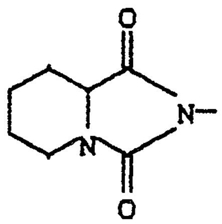 | H | $CH_3$ | $-CH-COOCH_3$<br>$CH_3$ | Fp 110° C |
| 9 | 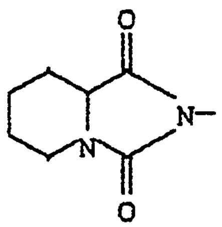 | F | $CH_3$ | $-CH_2-CH=CH_2$ | Fp 118° C |
| 10 | 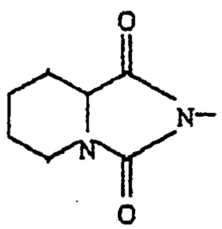 | F | $CH_3$ | $-CH(CH_3)_2$ | $^1$H-NMR*):<br>1,31 (d);<br>2,2 (s);<br>4,43 (m) |
| 11 | 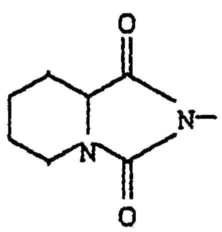 | F | $CH_3$ | $-CH-CH_3$<br>CN | Fp 59° C |
| 12 | 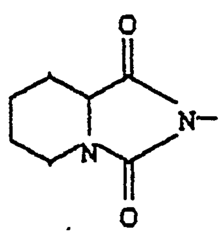 | F | $CH_3$ | $-CH-COOCH_3$<br>$CH_3$ | $^1$H-NMR*):<br>1,65 (d);<br>2,32 (s);<br>4,74 (q) |

| Bsp. Nr. | Het | $R^1$ | $R^2$ | $R^3$ | physikalische Eigenschaften |
|---|---|---|---|---|---|
| 13 | 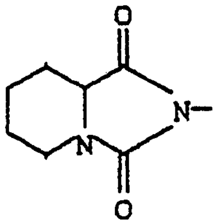 | F | $CH_3$ | 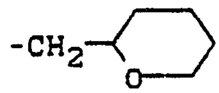 | $^1$H-NMR*): 2,23 (s); 4,23 (m) |
| 14 | 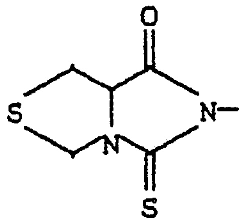 | F | $CH_3$ | $-CH_2-C{\equiv}CH$ | Fp 154° C |
| 15 | 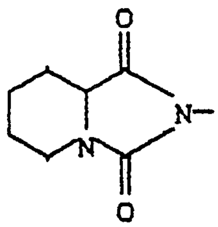 | F | $CH_3$ | 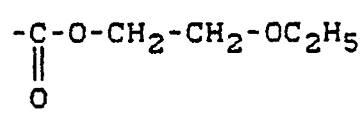 | $^1$H-NMR*): 1,21 (t); 2,24 (s) |
| 16 | 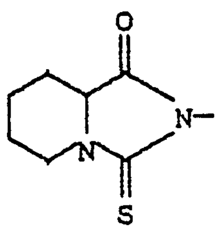 | F | $CH_3$ | $-CH_2-C{\equiv}CH$ | $^1$H-NMR*); 6,87 (d) |
| 17 | 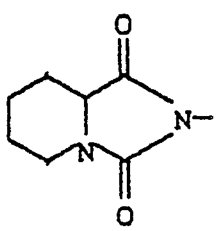 | F | $CH_3$ | $-CH_2-CH_2-OC_2H_5$ | $^1$H-NMR*): 6,70 (d) |

| Bsp. Nr. | Het | $R^1$ | $R^2$ | $R^3$ | physikalische Eigenschaften |
|---|---|---|---|---|---|
| 18 | 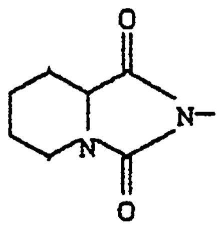 | F | $CH_3$ | $-CHF_2$ | $^1$H-NMR*): 6,48 (t) |
| 19 | 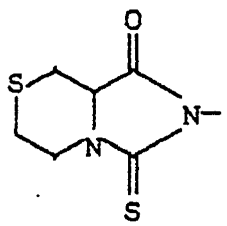 | H | $CH_3$ | $-CH(CH_3)-COOCH_3$ | Fp 98-99° C |
| 20 | 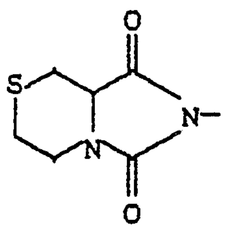 | H | $CH_3$ | $-CH_2-CH=CH_2$ | Fp 102° C |
| 21 | 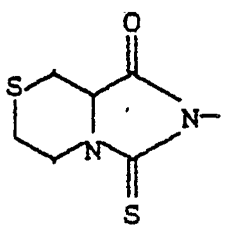 | H | $CH_3$ | $-CH_2-CH=CH_2$ | Fp 109° C |
| 22 | 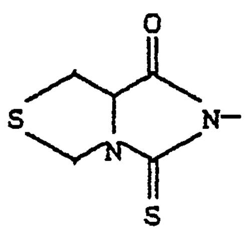 | H | $CH_3$ | $-CH(CH_3)-COOCH_3$ | Fp 80-82° C |

| Bsp. Nr. | Het | $R^1$ | $R^2$ | $R^3$ | physikalische Eigenschaften |
|---|---|---|---|---|---|
| 23 | 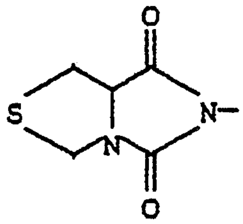 | H | $CH_3$ | $-CH_2-CH=CH_2$ | Fp 99° C |
| 24 | 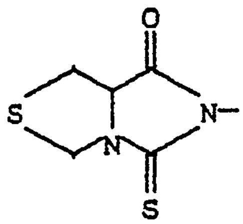 | H | $CH_3$ | $-CH_2-CH=CH_2$ | Fp 164-165° C |
| 25 | 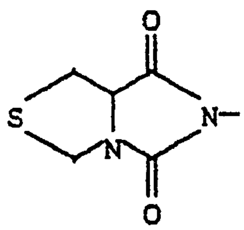 | H | $CH_3$ | $-CH_2-C \equiv CH$ | Fp 103-104° C |
| 27 | 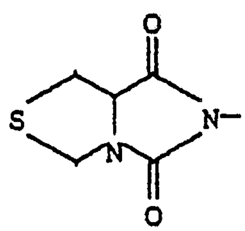 | F | $CH_3$ | $-CH_2$ | Fp 50° C |
| 28 | 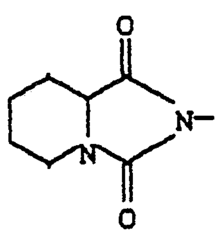 | F | $CH_3$ | $-CH_2-\underset{CH_3}{CH}-CH_2-O-C_2H_5$ | $^1$H-NMR*): 1,06 (d); 1,18 (t); 2,22 (s) |

| Bsp. Nr. | Het | $R^1$ | $R^2$ | $R^3$ | physikalische Eigenschaften |
|---|---|---|---|---|---|
| 29 | 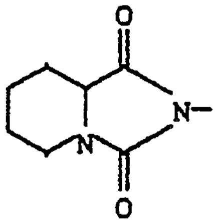 | F | $CH_3$ | $-(CH_2)_2-CH-OCH_3$<br>$CH_3$ | $^1$H-NMR*): 1,19 (d); 2,22 (s); 3,32 (s) |
| 30 | 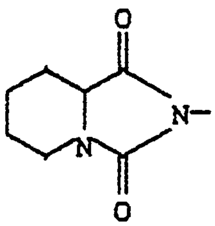 | F | $CH_3$ | $-CH_2-CH-CH_2-OCH_3$<br>$CH_3$ | $^1$H-NMR*): 1,05 (d); 2,22 (s); 3,32 (s) |
| 31 | 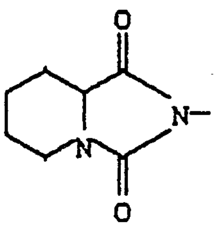 | F | $CH_3$ | $CH_2-OCH_3$<br>$-CH_2-C-CH_3$<br>$CH_2-OCH_3$ | $^1$H-NMR*): 1,06 (s); 3,32 (s); 3,35 (s) |
| 32 | 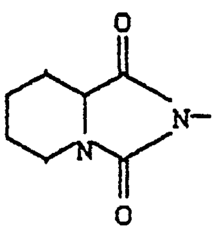 | F | $CH_3$ | $-CH_2-CH-CH_2-SCH_3$<br>$CH_3$ | $^1$H-NMR*): 1,13 (d); 2,1 (s); 3,87 (m) |
| 33 | 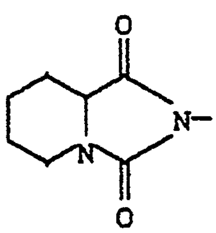 | F | $CH_3$ | $-C-NH-CH-CH_2-O-CH(CH_3)_2$<br>O $CH_3$ | Fp 65° C |

| Bsp. Nr. | Het | $R^1$ | $R^2$ | $R^3$ | physikalische Eigenschaften |
|---|---|---|---|---|---|
| 34 | 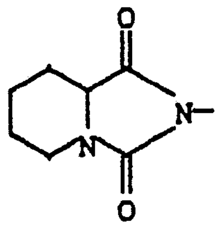 | F | $CH_3$ | 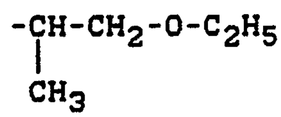 | $^1H$-NMR*): 1,18 (t); 1,29 (d); 4,4 (m) |
| 35 | 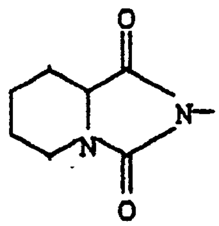 | F | $CH_3$ | 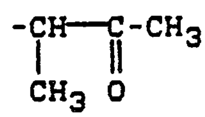 | Fp 86° C |
| 36 | 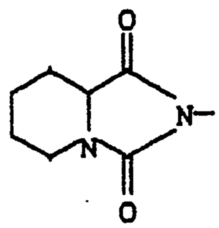 | F | $CH_3$ | 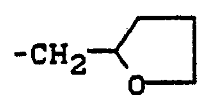 | $^1H$-NMR*): 2,23 (s); 6,68 (d); 6,99 (d) |
| 37 | 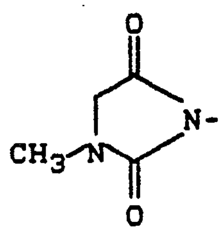 | F | $CH_3$ | $-CH(CH_3)_2$ | Fp 102-105° C |
| 38 | 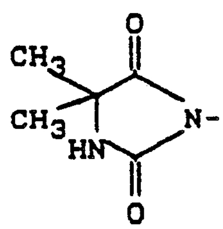 | F | $CH_3$ | $-CH(CH_3)_2$ | Fp 122-124° C |

| Bsp. Nr. | Het | $R^1$ | $R^2$ | $R^3$ | physikalische Eigenschaften |
|---|---|---|---|---|---|
| 39 | 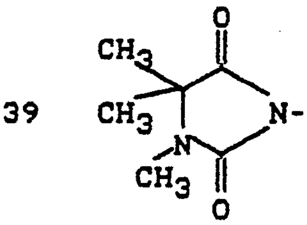 | F | $CH_3$ | $-CH(CH_3)_2$ | Fp 105-109° C |
| 40 | 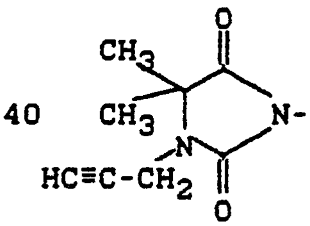 | F | $CH_3$ | $-CH(CH_3)_2$ | Öl |
| 41 | 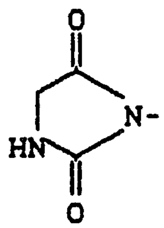 | F | $CH_3$ | $-CH(CH_3)_2$ | Fp 178-181° C |
| 42 | 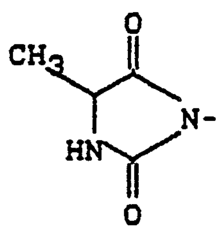 | F | $CH_3$ | $-CH(CH_3)_2$ | Fp 134-136° C |
| 43 | 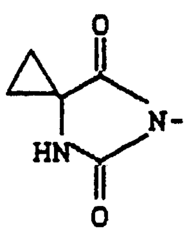 | F | $CH_3$ | $-CH(CH_3)_2$ | Fp 135-139° C |

| Bsp. Nr. | Het | $R^1$ | $R^2$ | $R^3$ | physikalische Eigenschaften |
|---|---|---|---|---|---|
| 44 | 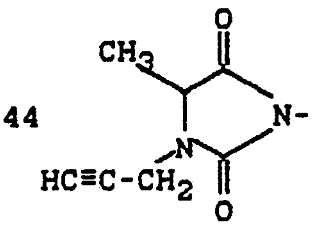 | F | $CH_3$ | $-CH(CH_3)_2$ | Öl |
| 45 | 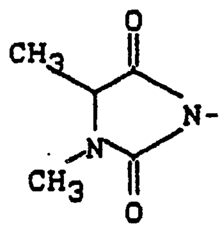 | F | $CH_3$ | $-CH(CH_3)_2$ | Fp 98-103° C |
| 46 | 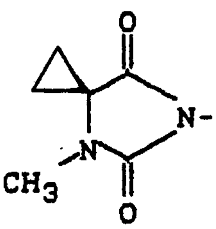 | F | $CH_3$ | $-CH(CH_3)_2$ | Fp 140-146° C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel II-1

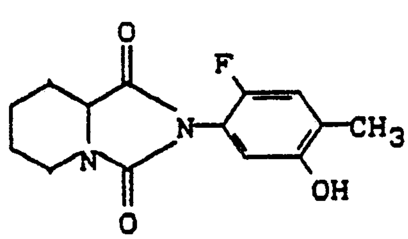

Zu 91 g (0,5 Mol) 5-Amino-4-fluor-2-methylphenol in 1000 ml Dichlormethan gibt man unter Rühren und Kühlung bei 0 °C bis 5 °C gleichzeitig 66,6 ml (0,52 Mol) Chlorameisensäurephenylester in 430 ml

Dichlormethan und 500 ml (0,5 Mol) 1N-Natronlauge, rührt nach beendeter Zugabe 15 Minuten nach, trennt dann die Dichlormethanphase ab, trocknet über Natriumsulfat und engt im Vakuum ein. Der Rückstand wird in 500 ml n-Propanol aufgenommen, mit 89 ml (0,5 Mol) Piperidin-2-carbonsäureethylester versetzt und so lange unter Rückfluß erhitzt, bis im Dünnschichtchromatogramm kein Ausgangsprodukt mehr nachweisbar ist (ca. 5 Stunden). Zur Aufarbeitung wird im Vakuum eingeengt, der Rückstand in Dichlormethan/ Essigester (6:1) aufgenommen und über Kieselgel filtriert. Nach Einengen im Vakuum wird aus Essigester/ Ether umkristallisiert.

Man erhält 85,7 g (62 % der Theorie) an 2-(2-Fluor-5-hydroxy-4-methylphenyl)-hexahydroimidazo[1,5-a]pyridin-1,3-dion vom Schmelzpunkt 162 °C.

Beispiel II-2

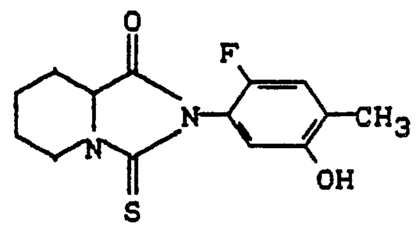

Zu einer Lösung von 6,6 g (0,036 Mol) 2-Fluor-5-hydroxy-4-methyl-phenylisothiocyanat in 100 ml Toluol (vgl. Bsp. X-1) gibt man tropfenweise unter Rühren 6,2 ml (0.039 Mol) Piperidin-2-carbonsäureethylester und rührt nach beendeter Zugabe 3 Stunden bei Raumtemperatur. Zur Aufarbeitung wird im Vakuum eingeengt und der ölige Rückstand durch Verrühren mit Diisopropylether zur Kristallisation gebracht.

Man erhält 10,2 g (98 % der Theorie) an 2-(2-Fluor-5-hydroxy-4-methylphenyl)-hexahydroimidazo[1,5a]-pyridin-1-on-3-thion vom Schmelzpunkt 90 °C - 93 °C.

Beispiel X-1

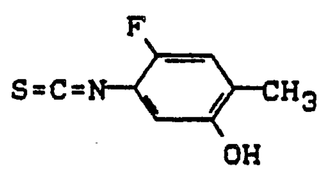

Zu einer Mischung aus 6,1 g (0,053 Mol) Thiophosgen und 40 ml Wasser gibt man bei 0 °C bis 5 °C eine Suspension von 5 g (0.036 Mol) 2-Fluor-5-hydroxy-4-methylanilin in 40 ml Chloroform, rührt 3 Stunden bei Raumtemperatur, trennt die organische Phase ab, trocknet über Magnesiumsulfat, filtriert, versetzt das Filtrat mit 50 ml trockenem Toluol und destilliert Chloroform und überschüssiges Thiophosgen ab. Die so erhältliche Toluollösung von 2-Fluor-5-hydroxy-4-methyl-phenylisothiocyanat wird ohne weitere Aufarbeitung in die nächste Reaktionsstufe eingesetzt.

Beispiel VIII-1

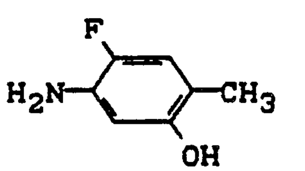

54 g (0,316 Mol) 4-Fluor-2-methyl-5-nitro-phenol in 800 ml Ethanol werden in Gegenwart von 3 g Platinoxid mit Wasserstoff bei 1 bis 5 bar und 20 °C bis 30 °C bis zur Konstanz der Wasserstoffaufnahme hydriert. Zur Aufarbeitung wird der Katalysator abfiltriert, das Lösungsmittel im Vakuum abdestilliert und der Rückstand aus Toluol umkristallisiert.

Man erhält 36,1 g (81 % der Theorie) an 5-Amino-4-fluor-2-methylphenol vom Schmelzpunkt 145 °C.

Beispiel XI-1

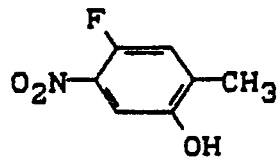

156 g (0,68 Mol) Methyl-(4-fluor-2-methyl-5-nitrophenyl)-carbonat werden in einer Mischung aus 400 ml Dioxan und 300 ml konzentrierter Salzsäure 12 Stunden auf Rückflußtemperatur erhitzt, anschließend abgekühlt, im Vakuum eingeengt und der Rückstand in Methyl-t-butylether aufgenommen. Man extrahiert mit 400 ml 10prozentiger Natronlauge, säuert die wässrige Phase an, filtriert den ausgefallenen Feststoff ab und kristallisiert aus Toluol um.

Man erhält 86 g (73,5 % der Theorie) an 4-Fluor-2-methyl-5-nitrophenol vom Schmelzpunkt 138 °C - 142 °C.

Beispiel XVII-1

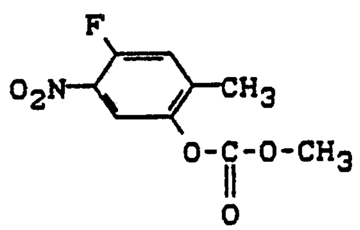

65 g (0,35 Mol) Methyl-(4-fluor-2-methyl-phenyl)-carbonat in 130 ml Dichlormethan werden bei 20 °C tropfenweise unter Rühren mit 65 g eines Gemisches aus konzentrierter Salpetersäure und konzentrierter Schwefelsäure (1:2) versetzt, anschließend 2 Stunden bei 20 °C und eine weitere Stunde bei 30 °C gerührt, abgekühlt, auf Eis gegossen, mit Dichlormethan extrahiert und destilliert.

Man erhält 62 g (76,7 % der Theorie) an Methyl-(4-fluor-2-methyl-5-nitro-phenyl)-carbonat vom Siedepunkt 120 °C - 125 °C bei 0,27 mbar und vom Schmelzpunkt 68 °C.

Beispiel XVII-2

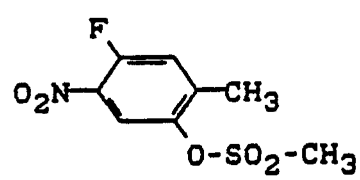

20 g (0,117 Mol) 4-Fluor-2-methyl-5-nitro-phenol in 40 ml Pyridin werden bei 10 °C unter Rühren tropfenweise mit 13,4 ml (0,117 Mol) Methansulfonsäurechlorid versetzt; anschließend wird das Reaktionsgemisch im Vakuum eingeengt, der Rückstand in Dichlormethan aufgenommen und nacheinander mit verdünnter Salzsäure und Wasser gewaschen, über Magnesiumsulfat getrocknet und im Vakuum vom Lösungsmittel befreit.

Man erhält 26,5 g (91 % der Theorie) an Methansulfonsäure-(4-fluor-2-methyl-5-nitro-phenyl)-ester vom Schmelzpunkt 96 °C - 97 °C.

Beispiel XVII-3

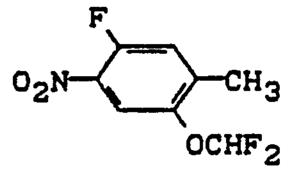

In eine Mischung aus 49 g (0,287 Mol) 4-Fluor-2-methyl-5-nitro-phenol und 45 ml Natronlauge (45 %ig) in 350 ml Dioxan wird bei 50 °C bis zur Sättigung Chlordifluormethan eingeleitet, anschließend wird eine Stunde bei Raumtemperatur gerührt, mit Eiswasser verdünnt und mit Ether extrahiert. Die vereinigten organischen Phasen werden mit Wasser gewaschen, über Natriumsulfat getrocknet und im Vakuum eingeengt.

Man erhält 58 g (91 % der Theorie) an 2-Difluormethoxy-5-fluor-4-nitro-toluol als Öl vom Brechungsindex $n_D^{20}$ = 1.5220.

Beispiel XVI-1

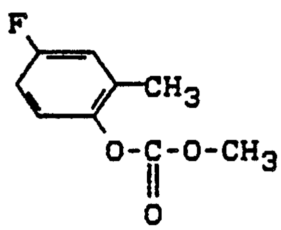

Zu 42 g (1,05 Mol) Natriumhydroxid in 660 ml Wasser gibt man 106 g (0,84 Mol) 2-Methyl-4-fluorphenol, tropft anschließend unter Rühren bei 5 °C 108 g (1,14 Mol) Chlorameisensäuremethylester zu, rührt nach beendeter Zugabe weitere 2 Stunden bei 5 °C, extrahiert mit Dichlormethan, trocknet über Natriumsulfat und destilliert im Vakuum.

Man erhält 139 g (88 % der Theorie) an Methyl-(4-fluor-2-methylphenyl)-carbonat vom Siedepunkt 98 °C bis 100 °C bei 16 mbar und vom Brechungsindex $n_D^{20}$ 1,4770.

Beispiel V-1

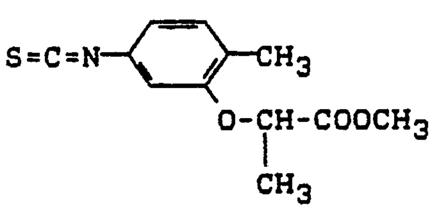

Zu einer Mischung von 1,15 ml (0,028 Mol) Thiophosgen in 60 ml Wasser gibt man bei 0 °C bis 5 °C

tropfenweise unter Rühren und Kühlung 3 g (0,014 Mol) 3-(1-Methoxycarbonylethoxy)-4-methylanilin in 60 ml Chloroform, läßt auf Raumtemperatur kommen, trennt die organische Phase ab, verdünnt mit 100 ml Toluol und destilliert überschüssiges Thiophosgen und Lösungsmittel zunächst unter Normaldruck, dann im Wasserstrahlvakuum ab.

Man erhält 2,88 g (82 % der Theorie) an 3-(1-Methoxycarbonylethoxy)-4-methyl-phenylisothiocyanat als Öl.

$^1$H-NMR ($CDCl_3$/Tetramethylsilan):

$\delta$ = 1,6 (d,3H); 2,3 (s,3H); 3,8 (s,3H); 4,8 (m,1H); 6,6 (m,1H), 6,8 (dd,1H) 7,4 (d,1H) ppm.

Beispiel V-2

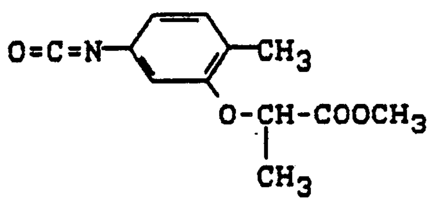

Zu 14,7 ml (0,028 Mol) einer 1,93 molaren Lösung von Phosgen in Toluol gibt man bei - 10 °C tropfenweise unter Rühren 3 g (0,014 Mol) 3-(1-Methoxycarbonylethoxy)-4-methylanilin in 30 ml Toluol, läßt nach beendeter Zugabe auf Raumtemperatur kommen, erhitzt dann auf Rückflußtemperatur und rührt 2 Stunden nach. Nach Entfernen des überschüssigen Phosgens durch Ausblasen mit Stickstoff wird das Lösungsmittel im Vakuum abdestilliert.

Man erhält 3,2 g (97 % der Theorie) an 3-(1-Methoxycarbonylethoxy)-4-methyl-phenylisocyanat als Öl.

$^1$H-NMR ($CDCl_3$/Tetramethylsilan): $\delta$ = 3,7 (s;3H) ppm.

In entsprechender Weise und gemäß den allgemeinen Angaben zur Herstellung erhält man die folgenden Iso(thio)cyanate der Formel (V):

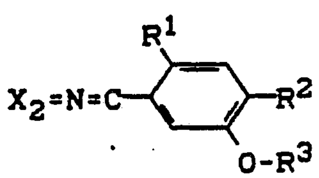 (V)

| Bsp. Nr. | $R^1$ | $R^2$ | $R^3$ | $X^2$ | physikalische Eigenschaften |
|---|---|---|---|---|---|
| V-3 | H | $CH_3$ | $-CH_2-CH=CH_2$ | O | $^1$H-NMR*): 6,1 (m) |
| V-4 | H | $CH_3$ | $-CH_2-C \equiv CH$ | O | $^1$H-NMR*): 2,5 (m) |
| V-5 | F | $CH_3$ | $-CH_2-C \equiv CH$ | O | 1H-NMR*): 2,5 (m) |
| V-6 | H | $CH_3$ | $-CH_2-CH=CH_2$ | S | $^1$H-NMR*): 6,1 (m) |
| V-7 | F | $CH_3$ | $-CH_2-C \equiv CH$ | S | $^1$H-NMR*): 2,5 (m) |
| V-8 | F | $CH_3$ | $-CH(CH_3)_2$ | O | Kp 68° C/ 0,2 mbar $n_D^{20}$ 1.504 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Beispiel XV-1

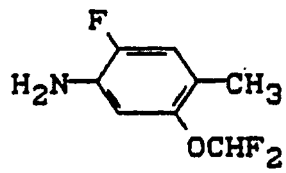

56 g (0,253 Mol) 5-Fluor-2-difluormethoxy-4-nitro-toluol und 10 g Platin auf Aktivkohle (1 %ig) in 400 ml Ethanol werden bei Raumtemperatur und Normaldruck bis zur Konstanz der Wasserstoffaufnahme hydriert. Zur Aufarbeitung wird der Katalysator abfiltriert und das Lösungsmittel im Vakuum entfernt.

Man erhält 42 g (86 % der Theorie) an 5-Difluormethoxy-2-fluor-4-methyl-anilin als Öl.

$^1$H-NMR ($DMSO-d_6$) : δ = 6,98 (t, 1H, $-OCHF_2$) ppm.

Beispiel XV-2

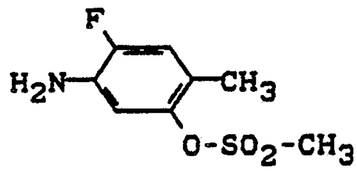

25 g (0,1 Mol) Methansulfonsäure-(4-fluor-2-methyl-5-nitrophenyl)-ester in 150 ml Ethoxyethanol werden mit 4 g Platin auf Aktivkohle (1 %ig) versetzt und bei Raumtemperatur und Normaldruck bis zur Konstanz der Wasserstoffaufnahme hydriert. Zur Aufarbeitung wird der Katalysator abfiltriert und das Lösungsmittel im Vakuum entfernt.

Man erhält 19,2 g (87,7 % der Theorie) an Methansulfonsäure-(4-fluor-2-methyl-5-amino-phenyl)-ester vom Schmelzpunkt 66 °C - 69 °C.

Beispiel XV-3

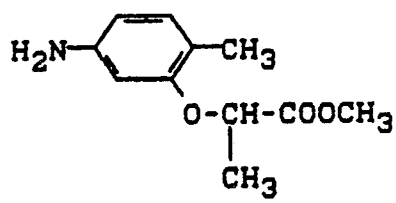

In eine Lösung aus 15 g (0,073 Mol) Essigsäure-N-[3-(1-methoxycarbonylethoxy)-4-methyl]-anilid in 250 ml Methanol wird 1 Stunde Chlorwasserstoff eingeleitet. Nach dem Abkühlen wird der ausgefallene Feststoff abgesaugt, in verdünnter wässriger Natriumhydrogencarbonatlösung gelöst und mit Essigester extrahiert ; die Essigesterlösung wird dann über Natriumsulfat getrocknet und im Vakuum eingeengt.

Man erhält 14,3 g (94 % der Theorie) an 3-(1-Methoxycarbonylethoxy)-4-methylanilin als Öl.

$^1$H-NMR (CDCl$_3$/Tetramethylsilan):

$\delta$ = 1,6 (d,3H); 2,2 (s,3H); 3,4 (s,2H); 3,7 (s,3H); 4,7 (q,1H); 6,1 (d,1H); 6,2 (dd,1H); 6,9 (dd,1H) ppm.

In entsprechender Weise und gemäß den allgemeinen Angaben zur Herstellung erhält man die folgenden Aniline der Formel (XV):

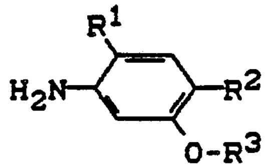

(XV)

| Bsp. Nr. | $R^1$ | $R^2$ | $R^3$ | physikalische Eigenschaften |
|---|---|---|---|---|
| XV-4 | H | $CH_3$ | $-CH_2-CH=CH_2$ | $^1$H-NMR*): 6,1 (m) |
| XV-5 | H | $CH_3$ | $-CH_2-C\equiv CH$ | $^1$H-NMR*): 2,5 (m) |
| XV-6 | F | $CH_3$ | $-CH_2-C\equiv CH$ | $^1$H-NMR*): 2,5 (m) |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

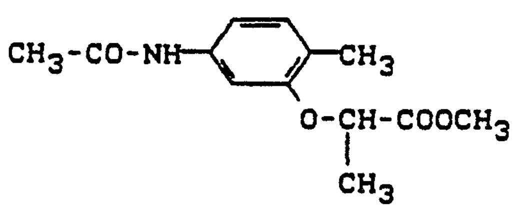

20 g (0.12 Mol) 5-Acetamido-2-methylphenol (Ber. dtsch. chem. Ges. 17, 609 [1884]), 30,1 g (0.18 Mol) 2-Brompropionsäuremethylester und 27,6 g (0,2 Mol) Kaliumcarbonat in 200 ml Aceton werden 6 Stunden auf Rückflußtemperatur erhitzt. Anschließend wird filtriert und das Filtrat im Vakuum eingeengt.

Man erhält 23,8 g (79 % der Theorie) an Essigsäure-N-[3-(1-methoxycarbonylethoxy)-4-methyl]-anilid ald Öl.

$^1$H-NMR ($CDCl_3$/Tetramethylsilan):

δ = 1,6 (d,3H); 2,1 (s,3H), 2,2 (s,3H); 3,7 (s,3H); 4,8 (q,1H); 6,8 (dd,1H); 7,0 (dd,1H); 7,2 (d,1H); 7,4 (s,1H) ppm.

In entsprechender Weise erhält man:

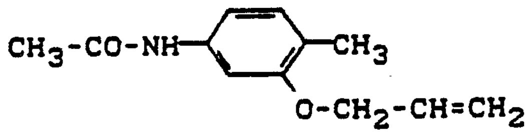

$^{1}H$-NMR*): 6,0 (m)

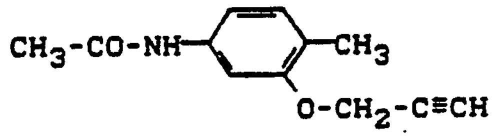

$^{1}H$-NMR*): 2,5 (m)

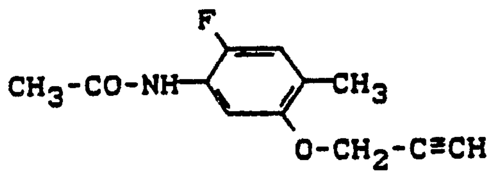

$^{1}H$-NMR*): 2,5 (m)

*) siehe Anmerkung zu den Beispielen der Formel XV.

Anwendungsbeispiele

In den folgenden Anwendungsbeispielen wurden die nachstehend aufgeführten Verbindungen als Vergleichsubstanzen eingesetzt:

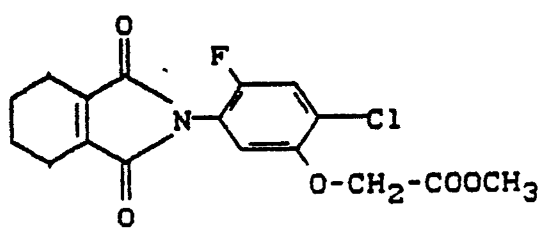

(A)

(bekannt aus EP-A 83 055 / Verbindung Nr. 1)

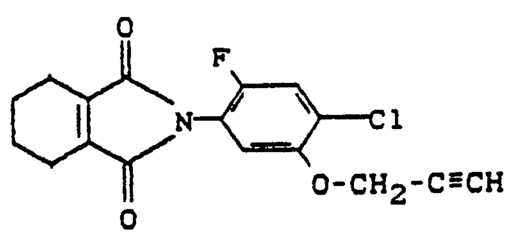

(B)

(bekannt aus EP-A 61 741 / Verbindung Nr. 10)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z.B. die Verbindung gemäß Herstellungsbeispiel 1.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test, z.B. die Verbindungen gemäß folgender Herstellungsbeispiele: 1 und 9.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
+ + starkes Austrocknen der Blätter, starker Blattfall
+ + + sehr starkes Austrocknen der Blätter, sehr starker Blattfall

Eine deutliche Überlegenheit im Vergleich zur unbehandelten Kontrolle zeigen in diesem Test beispielsweise die Verbindungen gemäß folgender Herstellungsbeispiele: 1, 5, 6, 9, 14, 19, 20, 22.

## Ansprüche

1. N-Aryl-Stickstoffheterocyclen der allgemeinen Formel (I),

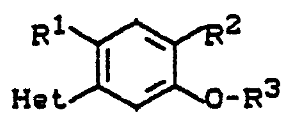

(I)

in welcher
Het für einen Heterocyclus der Formel

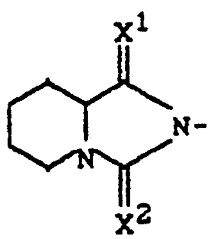

;

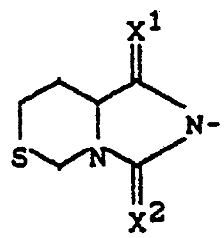

;

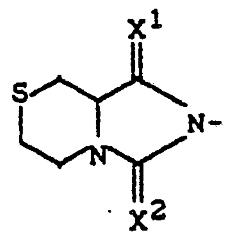

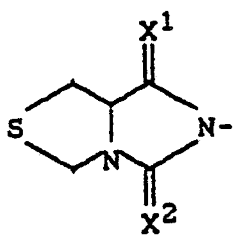

oder

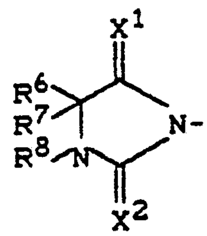

steht,

$R^1$ für Wasserstoff oder Halogen steht,
$R^2$ für Alkyl steht und
$R^3$ für jeweils gegebenenfalls substituiertes Alkyl, Alkenyl, Alkinyl oder Cycloalkyl steht oder für einen Rest
$-\underset{\underset{O}{\|}}{C}-R^4$
oder einen Rest $-SO_2-R^5$ steht,
wobei
$X^1$ und $X^2$ jeweils für Sauerstoff oder Schwefel stehen,
$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht,
$R^5$ für Alkyl, Halogenalkyl oder für gegebenenfalls substituiertes Aryl steht,
$R^6$ und $R^7$ entweder unabhängig voneinander jeweils für Wasserstoff, Alkyl, Alkenyl, Alkinyl oder Cycloalkyl stehen oder gemeinsam für einen zweifach verknüpften Alkandiylrest stehen und
$R^8$ für Wasserstoff, Alkyl, Alkenyl, Alkinyl, Alkoxyalkyl oder Cycloalkyl steht.

2. Verfahren zur Herstellung von N-Aryl-Stickstoffheterocyclen der allgemeinen Formel (I) gemäß Anspruch 1, dadurch gekennzeichnet, daß man

a) Phenolderivate der Formel (II),

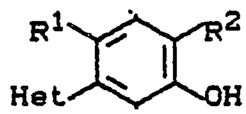

(II)

in welcher
$R^1$, $R^2$ und Het die in Anspruch 1 angegebene Bedeutung haben,
(α) mit Alkylierungsmitteln der Formel (IIIa),
$R^{3-1}$-$E^1$ (IIIa)
in welcher
$R^{3-1}$ für jeweils gegebenenfalls substituiertes Alkyl, Alkenyl, Alkinyl oder Cycloalkyl steht und
$E^1$ für eine elektronenanziehende Abgangsgruppe steht, oder
(β) mit Acylierungsmitteln der Formel (IIIb),
$R^4-\underset{\underset{O}{\|}}{C}-E^2$ (IIIb)
in welcher
$R^4$ die in Anspruch 1 angegebene Bedeutung hat und
$E^2$ für eine elektronenanziehende Abgangsgruppe steht, oder
(γ) mit Sulfonylierungsmitteln der Formel (IIIc),
$R^5$-$SO_2$-$E^3$ (IIIc)
in welcher
$R^5$ die oben angegebene Bedeutung hat und
$E^3$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt, oder daß man

b) N-Aryl-Stickstoffheterocyclen der allgemeinen Formel (Ia)

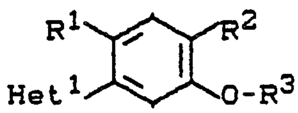

(Ia)

in welcher
$Het^1$ für einen Heterocyclus der Formel

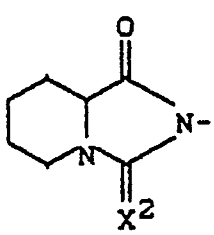 ; 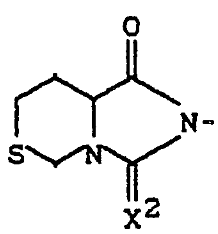 ; 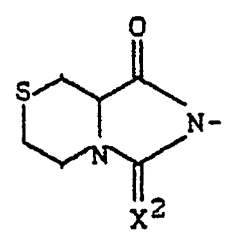 ;

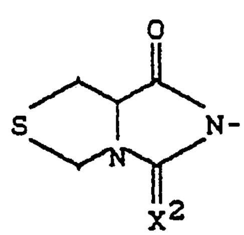 oder 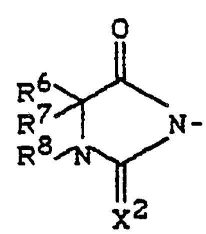

steht und
$R^1$, $R^2$, $R^3$, $R^6$, $R^7$, $R^8$ und $X^2$ die in Anspruch 1 angegebene Bedeutung haben,

erhält, wenn man Carbonsäureester der Formel (IV),
$R^9$-COOR$^{10}$ (IV)
in welcher
$R^9$ für einen Rest der Formel

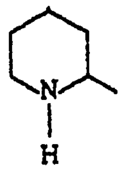 ; 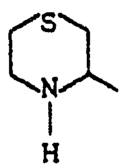 ; 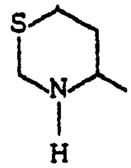 ; 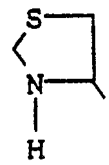

oder

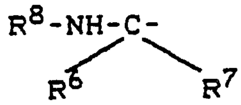

steht und
$R^{10}$ für Alkyl steht, wobei
$R^6$, $R^7$ und $R^8$ die Anspruch 1 angegebene Bedeutung haben,
oder deren Säureadditionssalze,
($\alpha$) mit Iso(thio)cyanaten der Formel (V),

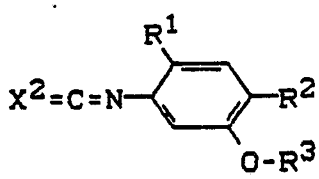 (V)

in welcher
$R^1$, $R^2$, $R^3$ und $X^2$ die in Anspruch 1 angegebene Bedeutung haben oder
($\beta$) mit Carbamaten der Formel (VI),

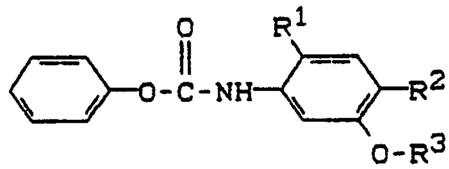 (VI)

in welcher
$R^1$, $R^2$ und $R^3$ die in Anspruch 1 angegebene Bedeutung haben,
gegebenenfalls in Gegenwart eines Verdünnungsmittels und gegebenenfalls in Gegenwart eines Reaktionshilfsmittels umsetzt, oder daß man

c) N-Aryl-Stickstoff-heterocyclen der allgemeinen Formel (Ib)

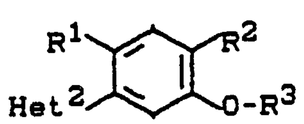 (Ib)

in welcher
Het$^2$ für einen Heterocyclus der Formel

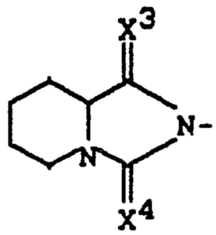 ; 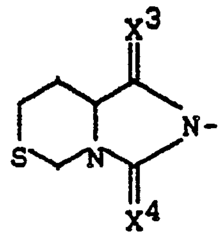 ; 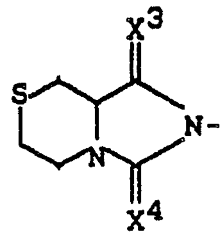 ;

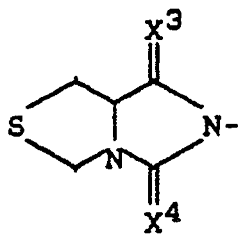 oder 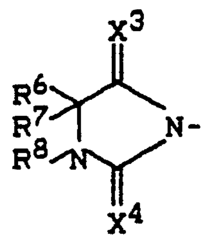 steht und

$R^1$, $R^2$, $R^3$, $R^6$, $R^7$ und $R^8$ die oben angegebene Bedeutung haben,
wobei
$X^3$ und $X^4$ jeweils für Sauerstoff oder Schwefel stehen, mit der Bedingung, daß mindestens einer der Reste $X^3$ oder $X^4$ für Schwefel steht,
erhält, wenn man
N-Arylstickstoffheterocyclen der Formel (Ia),

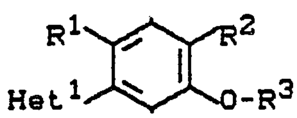 (Ia)

in welcher
$Het^1$ für einen Heterocyclus der Formel

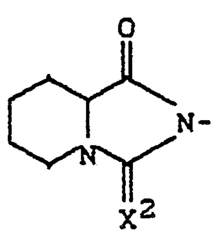 ; 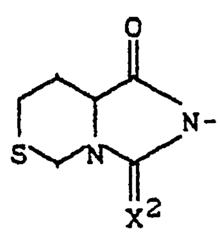 ; 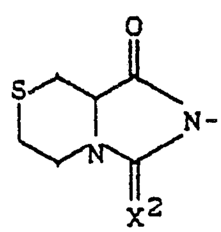

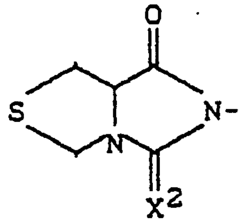 oder 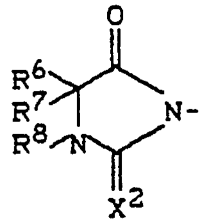 steht und

$R^1$, $R^2$, $R^3$, $R^6$, $R^7$ und $R^8$ die in Anspruch 1 angegebene Bedeutung haben,
mit "Lawessons-Reagenz" der Formel (VII)

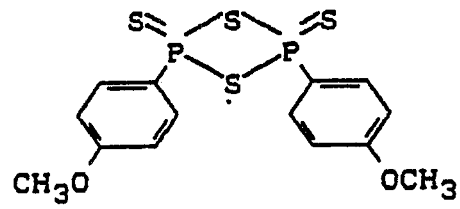

(VII)

gegebenenfalls in Gegenwart eines Verdünnungsmittels umsetzt.

3. Herbizide und pflanzenwuchsregulierende Mittel, gekennzeichnet durch einen Gehalt an mindestens einem N-Aryl-Stickstoffheterocyclus der Formel (I) gemäß Anspruch 1.

4. Verfahren zur Bekämpfung von Unkräutern, dadurch gekennzeichnet, daß man N-Aryl-Stickstoffheterocyclen der Formel (I) gemäß Anspruch 1 auf die Unkräuter und/oder ihren Lebensraum einwirken läßt.

5. Verwendung von N-Aryl-Stickstoffheterocyclen der Formel (I) gemäß Anspruch 1 zur Bekämpfung von Unkräutern und/oder als Pflanzenwuchsregulatoren.

6. Verfahren zur Herstellung von herbiziden und/oder pflanzenwuchsregulierenden Mitteln, dadurch gekennzeichnet, daß man N-Aryl-Stickstoffheterocyclen der Formel (I) gemäß Anspruch 1 mit Streckmitteln und/oder oberflächenaktiven Substanzen vermischt.

7. Phenolderivate der Formel (II)

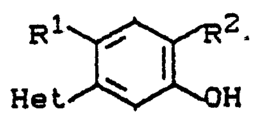

(II)

in welcher
$R^1$ für Wasserstoff oder Halogen steht,
$R^2$ für Alkyl steht und
Het für einen Heterocyclus der Formel

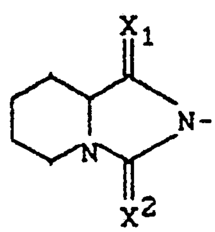 ; 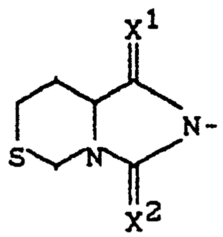 ; 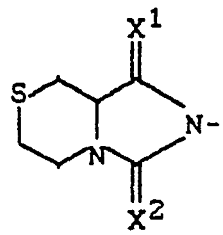 ;

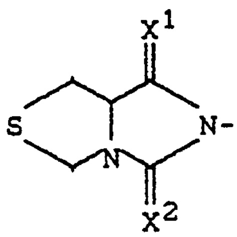 oder 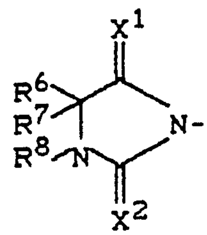 steht,

wobei
$X^1$ und $X^2$ jeweils für Sauerstoff oder Schwefel stehen,
$R^6$ und $R^7$ entweder unabhängig voneinander jeweils für Wasserstoff, Alkyl, Alkenyl, Alkinyl oder Cycloalkyl stehen oder gemeinsam für einen zweifach verknüpften Alkandiylrest stehen und
$R^8$ für Wasserstoff, Alkyl, Alkenyl, Alkinyl, Alkoxyalkyl oder Cycloalkyl steht.

8. Iso(thio)cyanate der Formel (Va)

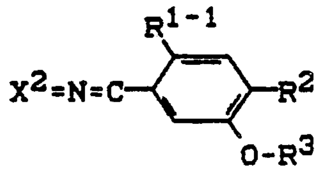

(Va)

in welcher
$X^2$ für Sauerstoff oder Schwefel steht,
$R^{1-1}$ für Halogen steht,
$R^2$ für Alkyl steht,
$R^3$ für jeweils gegebenenfalls substituiertes Alkyl, Alkenyl, Alkinyl oder Cycloalkyl oder für einen Rest
- C -$R^4$
||
O
oder einen Rest -$SO_2$-$R^5$ steht, wobei
$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht und
$R^5$ für Alkyl, Halogenalkyl oder für gegebenenfalls substituiertes Aryl steht.

9. Carbamate der Formel (VI)

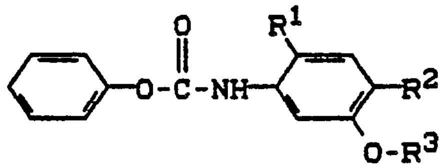

(VI)

in welcher
$R^1$ für Wasserstoff oder Halogen steht,
$R^2$ für Alkyl steht und
$R^3$ für jeweils gegebenenfalls substituiertes Alkyl, Alkenyl, Alkinyl oder Cycloalkyl steht oder für einen Rest
- C -$R^4$
||
O
oder einen Rest -$SO_2R^5$ steht,
wobei
$R^4$ für Alkyl, Alkoxyalkyl, Halogenalkyl, Alkoxy, Alkoxyalkoxy, Halogenalkoxy, Halogenalkoxyalkoxy oder Alkoxyalkylamino steht und
$R^5$ für Alkyl, Halogenalkyl oder für gegebenenfalls substituiertes Aryl steht.

10. Aniline der Formel (XVb)

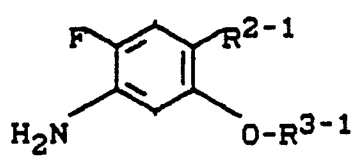

(XVb)

in welcher
$R^{2-1}$ für Alkyl steht und
$R^{3-1}$ -CO-$R^4$ steht, wobei
$R^{4-1}$ für Alkoxyalkyl oder Alkoxyalkylamino steht.

Europäisches Patentamt

# EUROPÄISCHER RECHERCHENBERICHT

Nummer der Anmeldung

EP 89114065.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 5) |
|---|---|---|---|
| X | EP - A2/A3 - 0 070 389 (SUMITOMO) * Zusammenfassung; Ansprüche 1,9,10,12,14; Seite 6 * -- | 1-8,10 | C 07 D 471/04<br>C 07 D 513/04<br>C 07 D 233/86<br>C 07 C 265/12<br>C 07 C 331/28<br>C 07 C 271/58<br>C 07 C 211/46<br>A 01 N 43/50<br>A 01 N 43/78<br>A 01 N 43/84<br>A 01 N 43/86<br>//(C 07 D 471/04,<br>C 07 D 221:00,<br>C 07 D 209:00) |
| X | EP - A1 - 0 104 532 (NIPPON) * Zusammenfassung; Seiten 2,3 * -- | 1-6,8 | |
| X | EP - A1 - 0 272 594 (HOECHST) * Ansprüche 1-6 * -- | 1-8 | |
| X | US - A - 4 179 276 (CHENG) * Zusammenfassung; Formel Ia; Spalte 3, Zeilen 63,64 * -- | 1-8 | |
| X | GB - A - 1 503 244 (MITSUBISHI) * Seite 1, Zeile 36 - Seite 2, Zeile 5; Seite 3, Zeilen 11-21; Anspruch 49 * -- | 1-8 | **RECHERCHIERTE SACHGEBIETE (Int. Cl. 5)** |
| X | US - A - 4 138 242 (GODDARD) * Spalte 2, Zeilen 28-45; Spalte 3; Anspruch 1 * -- | 1-6,8 | C 07 D 471/00<br>C 07 D 513/00<br>C 07 D 233/00<br>C 07 C 265/00<br>C 07 C 331/00<br>C 07 C 271/00<br>C 07 C 211/00 |
| X | DE - A - 1 445 797 (HOECHST) * Seiten 1,2 * -- | 1,2,7,8 | |
| A | DE - A - 1 593 523 (SCHERING) * Seite 3, Zeile 8 (Formel) * -- | 9 | |
| A | CHEMICAL ABSTRACTS, Band 71, Nr. 3, 21. Juli 1969, | 9 | |

Der vorliegende Recherchenbericht wurde für alle Patentansprüche erstellt.

| Recherchenort | Abschlußdatum der Recherche | Prüfer |
|---|---|---|
| WIEN | 18-10-1989 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-
EP 89114065.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | Columbus, Ohio, USA<br>C.H. MACK et al. "Synthesis of some phenyl N-aryl-carbamates"<br>Seite 285, Spalte 2, Zusammenfassung-Nr. 12 695j<br>& J.Chem.Eng.Data 1969, 14(2), 258-61<br>---- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prufer |
|---|---|---|
| WIEN | 18-10-1989 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument